(19) Europäisches Patentamt
European Patent Office
Office européen des brevets

(11) Veröffentlichungsnummer: **0 040 177**
**B1**

# (12) EUROPÄISCHE PATENTSCHRIFT

(45) Veröffentlichungstag der Patentschrift: **20.07.83**

(21) Anmeldenummer: **81810178.4**

(22) Anmeldetag: **07.05.81**

(51) Int. Cl.³: **C 07 C 67/347,** C 07 C 69/73, C 07 C 79/46, C 07 C 121/75, C 07 C 121/76, C 07 C 121/70, C 07 C 103/76, C 07 C 45/68, C 07 C 49/217

## (54) Verfahren zur Herstellung von Alkenylbenzol- oder -naphthalincarbonsäurederivaten.

(30) Priorität: **13.05.80 CH 3732/80**

(43) Veröffentlichungstag der Anmeldung: **18.11.81 Patentblatt 81/46**

(45) Bekanntmachung des Hinweises auf die Patenterteilung: **20.07.83 Patentblatt 83/29**

(84) Benannte Vertragsstaaten: **BE CH DE FR GB LI**

(56) Entgegenhaltungen:
**DE-A-2 041 563**
**US-A-3 527 794**

**CHEMICAL ABSTRACTS, Band 92, Nr. 13, 31. März 1980, Seite 593, Zusammenfassung 110 202z, Columbus, Ohio, USA. A. BIAVATI et al.: "Palladium or nickel-catalyzed benzoylation and phenylation of methyl acrylate"**

(73) Patentinhaber: **CIBA-GEIGY AG, Patentabteilung Postfach, CH-4002 Basel (CH)**

(72) Erfinder: **Blaser, Hans-Ulrich, Dr., Brücklismattstrasse 18, CH-4107 Ettingen (CH)**
Erfinder: **Reinehr, Dieter, Dr., Wolfsheule 10, D-7842 Kandern (DE)**
Erfinder: **Spencer, Alwyn, Dr,, Schützengraben 15, CH-4051 Basel (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

# Verfahren zur Herstellung von Alkenylbenzol- oder -naphthalincarbonsäurederivaten

Die vorliegende Erfindung betrifft ein neues Verfahren zur Herstellung von Alkenylbenzol- oder -naphthalincarbonsäurederivaten.

Aus der US-Patentschrift 3 922 299 ist bekannt, dass man vinylisch oder allylisch substituierte organische Verbindungen, besonders Zimtsäure und Zimtsäureester durch katalytische Umsetzung von entsprechenden Halogeniden mit aktivierten Olefinen, wie Acrylsäuremethylester, in Gegenwart von tertiären Aminen herstellen kann. Als Katalysatoren werden bevorzugt Gemische von Palladiumacetat und Triphenylphosphin oder Tri-(orthotolyl)-phosphin verwendet. Die Umsetzung kann auch so vorgenommen werden, dass man zuerst aus dem Halogenid und dem Katalysatorsystem einen Komplex bildet und diesen anschliessend in Gegenwart eines tertiären Amins mit dem Olefin reagieren lässt. Andererseits ist bekannt, dass die Umsetzung von Benzoylchlorid mit Acrylsäuremethylester in Gegenwart stöchiometrischer Mengen eines Nickel(O)-Katalysators bei Nachbehandlung des Reaktionsgemisches mit Jod in Methanol zur Bildung von trans-3-Benzoylacrylsäuremethylester führt. Als Nebenprodukt fällt dabei Zimtsäuremethylester an. Durch Umsetzung eines Komplexes aus Benzoylpalladiumchlorid und Triphenylphosphin mit Acrylsäuremethylester bei 70–85°C in Gegenwart von Triäthylamin erhält man Zimtsäuremethylester als Hauptprodukt und Benzoylacrylsäuremethylester als Nebenprodukt. Werden das Palladium und das Triphenylphosphin nur in katalytischen Mengen eingesetzt, so verschiebt sich das Reaktionsgleichgewicht zugunsten der Bildung des Benzoylacrylsäuremethylesters (Gewichtsverhältnis von Benzoylacrylsäuremethylester:Zimtsäuremethylester = ca. 8,3:1) [vgl. Transition Met. Chem. 2,270 (1977) und 4,398 (1979)]. Schliesslich ist aus Synthesis, 777 (1977) bekannt, dass die Umsetzung von aromatischen Säurehalogeniden mit 1-Alkinen unter Pd-Katalyse ohne Decarbonylierung zu Alkinylketonen führt.

Es wurden nun gefunden, dass man Verbindungen der Formel I

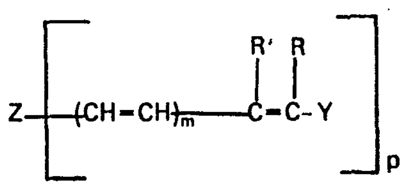 (I),

worin m Null oder die Zahl 1,
p die Zahl 1 oder 2,
Z bei p = 1 gegebenenfalls substituiertes Phenyl oder Naphthyl und p = 2 gegebenenfalls substituiertes Phenylen oder Naphthylen,
R Wasserstoff, $C_{1-4}$-Alkyl, $-CH_2COOR''$ oder $-CH_2CH_2CN$ und
R′ Wasserstoff, oder bei p = 1 und m = Null auch $C_{1-4}$-Alkyl, -CN oder -COOR″ bedeuten, wobei mindestens eines von R und R′ Wasserstoff sein muss,
Y -CN, -COOR″, $-CON(R'')_2$ oder -COR″ und die R″ unabhängig voneinander $C_1$-$C_{12}$-Alkyl oder Phenyl darstellen, dadurch herstellen kann, dass man eine Verbindung der Formel II

$$Z-\left[(CH=CH)_m-CO-X\right]_p \quad \text{(II)},$$

worin Z, m und p die unter Formel I angegebene Bedeutung haben und X Chlor, Brom oder Jod darstellt, in Gegenwart einer Base und unter Zusatz von Palladium-Metall oder von Palladiumverbindungen, die unter den Reaktionsbedingungen phosphorfreie labile Palladium(O)Verbindungen bilden, als Katalysator mit einer Verbindung der Formel III oder, bei p = 2, auch einem Gemisch von zwei verschiedenen Verbindungen der Formel III

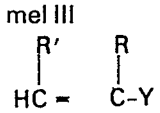 (III),

worin für R, R′ und Y das unter Formel I Angegebene gilt, umsetzt.

Nach dem erfindungsgemässen Verfahren lassen sich die Verbindungen der Formel I auf einfache wirtschaftliche Weise und unter Verwendung von leicht zugänglichen Ausgangsprodukten herstellen. Dabei ist es überraschend, dass die Reaktion selektiv unter Decarbonylierung der Säurehalogenide der Formel II verläuft.

Die in Gruppen Z vorkommenden Substituenten sind solche, die unter den Reaktionsbedingungen inert sind. die genannten Gruppen Z können ein- oder mehrfach substituiert sein. Dabei können die Substituenten gleich oder verschieden sein.

Als Substituenten an Gruppen Z kommen z.B. in Betracht: Halogenatome, Formyl, $-CH(OCH_3)_2$, $-CH(OC_2H_5)_2$,

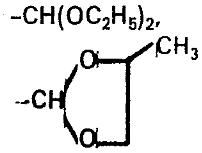 , 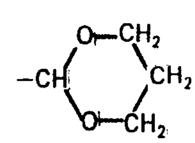 ,

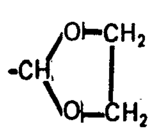 ,

$C_{1-10}$-Alkyl-, $C_{1-16}$-Alkoxy, Phenoxy-, Di($_{1-10}$-alkyl)amino-, Nitro-, Cyano-, Chlormethyl-, Trifluormethyl-, Benzyl-, $C_{1-4}$-Alkylsulfonyl-, -CO-$C_{1-10}$-Alkyl-, -CO-Phenyl-, 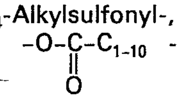 -Alkyl-, -COO-$C_{1-10}$-Alkyl-, -COO-Phenyl-, Phenyl- oder Naphthylgruppen, die ihrerseits durch Halogenatome, $C_{1-10}$-Alkyl-, $C_{1-10}$-Alkoxy-, Di($C_{1-10}$-alkyl)amino-, Nitro-, Cyano-, Trifluormethyl-, -CO-

$C_{1-10}$-Alkyl-, -CO-Phenyl-, -COO-$C_{1-10}$-Alkyl- oder -COO-Phenylgruppen substituiert sein können.

Phenyl- und Naphthylsubstituenten an Gruppen Z sind bevorzugt monosubstituiert oder unsubstituiert. Alkylgruppen R″ sowie Alkyl- und Alkoxygruppen in den oben erwähnten Substituenten können geradkettig oder verzweigt sein und weisen bevorzugt 1-8 und insbesondere 1-4 C-Atome auf. Halogensubstituenten sind z.B. Fluor, Chlor oder Brom. Als Beispiele definitionsgemässer Gruppen R″ bzw. Substituenten an Gruppen Z seien erwähnt: die Methyl-, Äthyl-, n-Propyl-, Isopropyl-, n-, sek- und tert-Butyl-, n-Pentyl-, 2-Pentyl-, n-Hexyl-, n-Heptyl, n-Octyl- und n-Decylgruppe; die Methoxy-, Äthoxy-, n-Propoxy-, n-Butoxy-, n-Hexyloxy- und n-Decyloxygruppe; die N,N-Dimethylamino-, N,N-Diäthylamino-, N,N-Di-n-propylamino-, N,N-Di-n-butylamino-, N,N-Di-n-hexylamino-, N,N-Di-n-octylamino-, N-Methyl-N-äthylamino-, N-Methyl-N-n-propylamino-, N-Äthyl-N-n-hexylamino- und N-Äthyl-N-n-butylaminogruppe; die Methyl- und Äthylsulfonylgruppe; die Acetyl-, Propionyl-, Butyryl-, Valeroyl- und Octanoylgruppe; die Carbonsäure-methyl, -äthyl-, -n-propyl-, -isopropyl-, -n-butyl-, -n-pentyl-, -n-hexyl-, n-heptyl- und -n-decylestergruppe.

Alkylgruppen R und R′ sind bevorzugt geradkettig und weisen ein oder zwei C-Atome auf. X in Formel II stellt vorzugsweise Chlor dar.

Als Verbindungen der Formel II kommen insbesondere in Betracht:

1. Verbindungen der Formel IIa

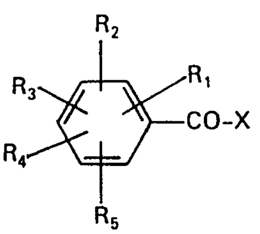

(IIa),

worin X Chlor oder Brom,

$R_1$ Wasserstoff, Cl, Br, F, Formyl, $-CH(OCH_3)_2$, $-CH(OC_2H_5)_2$,

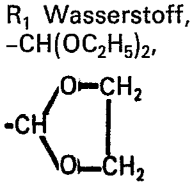

,

$C_{1-4}$-Alkyl, $C_{1-4}$-Alkoxy, Phenoxy, Di($C_{1-2}$-alkyl)amino, $-NO_2$, -CN, $-CF_3$, $-CH_2Cl$, $C_{1-4}$-Alkylsulfonyl, Benzyl, -CO-$C_{1-4}$-Alkyl, -CO-Phenyl, -OCO-$C_{1-4}$-Alkyl, -COO-$C_{1-4}$-Alkyl, -COO-Phenyl, Phenyl, Chlorphenyl, Bromphenyl, Methylphenyl, Methoxyphenyl, 1- oder 2-Naphthyl, $R_2$ und $R_3$ unabhängig voneinander Wasserstoff, Cl, Br, F, $-NO_2$, $C_{1-4}$-Alkyl oder $C_{1-4}$-Alkoxy, besonders Methyl oder Methoxy, $R_4$ und $R_5$ Wasserstoff oder, wenn $R_1$, $R_2$, und $R_3$ je Chlor, Brom, Fluor oder Methyl sind, ebenfalls je Chlor, Brom, Fluor oder Methyl bedeuten. Bevorzugt sind Verbindungen der Formel IIa, worin X Chlor, $R_1$ Wasserstoff, Cl, Br, F, J, Chlormethyl, $C_{1-4}$-Alkyl, besonders Methyl oder Äthyl, Methoxy-, $-OCOCH_3$, $-COOCH_3$, $-NO_2$, -CN, Formyl, Methylsulfonyl oder Phenyl, $R_2$ Wasserstoff, Cl, Br, F, Methyl, Äthyl, Methoxy oder Nitro, $R_3$ Wasserstoff, Cl, Br, F, Methyl, Äthyl oder Methoxy und $R_4$ und $R_5$ je Wasserstoff oder, bei $R_1$-$R_3$ = Cl oder F, ebenfalls Cl oder F bedeuten.

2. Verbindungen der Formel IIb

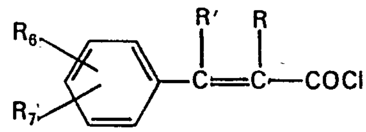

(IIb),

worin eines von R und R′ Wasserstoff und das andere Wasserstoff oder Methyl oder R Wasserstoff und R′ -COO-Alkyl mit 1-4 C-Atomen im Alkyl, $R_6$ Wasserstoff, Cl, Br, F, $-NO_2$, -CN, $-SO_2CH_3$, Methyl, Äthyl, Methoxy, Äthoxy, -CHO oder $-CH(OCH_3)_2$ und $R_7$ Wasserstoff, Cl, Br, F, $-NO_2$, Methyl, Äthyl, Methoxy oder Äthoxy darstellen. Bevorzugte Verbindungen der Formel IIb sind solche, worin $R_6$ Wasserstoff, Methyl, Methoxy, Cl, Br, F, $-NO_2$ oder -CHO, $R_7$ Wasserstoff und eines von R und R′ Wasserstoff und das andere Wasserstoff oder Methyl oder R Wasserstoff und R′ $-COOCH_3$ oder $-COOC_2H_5$ bedeuten.

3. Verbindungen der Formel IIc

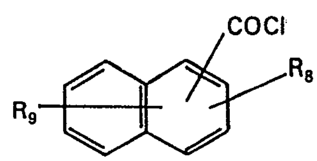

(IIc),

worin die Gruppe -COCl in 1- oder 2-Stellung gebunden ist, $R_8$ und $R_9$ an denselben Ring oder an verschiedene Ringe gebunden sein können, $R_8$ Wasserstoff, Cl, Br, F, Methyl, Äthyl, Methoxy, Äthoxy, -CHO, $-COCH_3$, $-SO_2CH_3$, -CN, $-NO_2$ oder $-CH(OCH_3)_2$ und $R_9$ Wasserstoff, Cl, Br, F, Methyl, Methoxy oder $-NO_2$ bedeuten. Als Verbindungen der Formel IIc werden solche bevorzugt, worin $R_8$ Methyl und insbesondere Wasserstoff und $R_9$ Wasserstoff darstellen.

4. Verbindungen der Formel IId

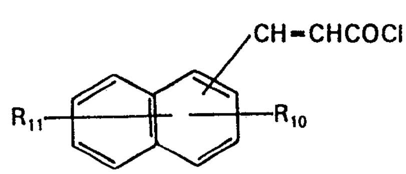

(IId),

worin die Gruppe -CH=CHCOCl in 1- oder 2-Stellung gebunden ist, $R_{10}$ und $R_{11}$ an denselben Ring oder an verschiedene Ringe gebunden sein kön-

nen, $R_{10}$ Wasserstoff, Cl, Br, F, Methyl, Methoxy, $-NO_2$, -CHO, -CN, $-SO_2CH_3$ oder $-CH(OCH_3)_2$ und $R_{11}$ Wasserstoff, Cl, Br, F, Methyl, Methoxy oder $-NO_2$ bedeuten. Bevorzugte Verbindungen der Formel IId sind solche, worin $R_{10}$ Methyl und insbesondere Wasserstoff und $R_{11}$ Wasserstoff darstellen.

5. Verbindungen der Formel IIe

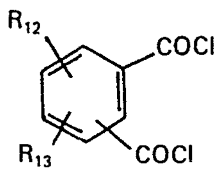

(IIe),

worin $R_{12}$ Wasserstoff, -CO-Phenyl, Cl, Br, F, -CN, -CHO, $-NO_2$ oder Methyl und $R_{13}$ Wasserstoff, Cl, Br, F oder Methyl bedeuten. Als Verbindungen der Formel IIe werden Iso- und Terephthalsäuredichlorid, die gegebenenfalls durch eine Methyl- oder $NO_2$-Gruppe substituiert sind, und vor allem das unsubstituierte Iso- oder Terephthalsäuredichlorid, bevorzugt.

6. Verbindungen der Formel IIf

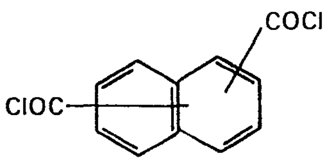

(IIf),

wobei die -COCl-Gruppen an denselben Ring oder an verschiedene Ringe gebunden sein können. Als Verbindungen der Formel IIf werden 1,4- und 2,6-Naphthalindicarbonsäuredichlorid bevorzugt.

7. Verbindungen der Formel IIg

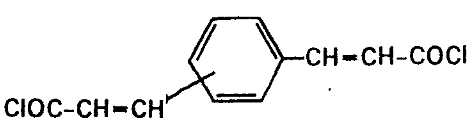

(IIg),

wobei die -CH=CH-COCl-Gruppen bevorzugt in 1,3- oder 1,4-Stellung gebunden sind.

8. Verbindungen der Formel IIh

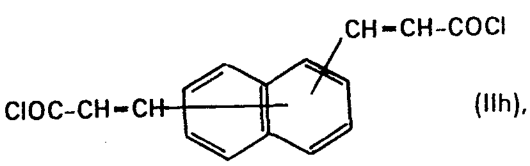

(IIh),

wobei die -CH=CH-COCl-Gruppen an denselben Ring oder an verschiedene Ringe und bevorzugt in 1,4- oder 2,6-Stellung gebunden sind.

Bevorzugte Bedeutungen von R und R′ sind: R und R′ = Wasserstoff, R = Wasserstoff und R′ = Methyl, -CN oder -COOR″ (p = 1) oder R′ = Wasserstoff und R = Methyl, $-CH_2COOR''$ oder $-CH_2CH_2CN$, wobei R″ Alkyl mit 1-4 und insbesondere 1 oder 2 C-Atomen darstellt. Y ist vorzugsweise -CN, -COR″, -COOR″ oder $-CON(R'')_2$, worin R″ Alkyl mit 1-4 und insbesondere 1 oder 2 C-Atomen bedeuten.

Als Verbindungen der Formel I mit p = 2 werden symmetrische Verbindungen bevorzugt, d.h. solche, worin R und Y in den beiden Gruppen —CH=C(R)-Y bzw. —CH=CH-CH=C(R)-Y (R′ = H) dieselbe Bedeutung haben.

Besonders bevorzugt verwendet man als Säurehalogenide Verbindungen der Formel IIa, worin X Chlor, eines von R und R′ Wasserstoff und das andere Wasserstoff oder Methyl oder R Wasserstoff und R′ -COO-Alkyl mit 1-4 C-Atomen im Alkyl, $R_1$ Wasserstoff, Cl, Br, F, J, Formyl, Methyl, Methoxy, Chlormethyl, Cyano, Nitro, $-OCOCH_3$, $-COOCH_3$ oder Phenyl, $R_2$ Wasserstoff, Cl, F, Methyl oder Methoxy, $R_3$ Wasserstoff, Cl, F oder Methoxy und $R_4$ und $R_5$ je Wasserstoff, oder bei $R_1$ bis $R_3$ = Cl oder F, ebenfalls Cl oder F bedeuten; Verbindungen der Formel IIb worin $R_6$ Wasserstoff, Chlor oder Nitro und $R_7$ Wasserstoff sind; Verbindungen der Formel IIc, worin die Gruppe -COCl in 1- oder 2-Stellung gebunden ist, $R_8$ Methyl und insbesondere Wasserstoff und $R_9$ Wasserstoff darstellen, Iso- oder Terephthalsäuredichlorid, 1,4- oder 2,6-Naphthalindicarbonsäuredichlorid. Ganz besonders bevorzugt sind 4-Formylbenzoylchlorid, 4-Brombenzoylchlorid und vor allem Benzoylchlorid.

Bevorzugte Verbindungen der Formel III sind solche, worin
-R und R′ Wasserstoff und Y -CN, -COR″, -COOR″ oder $-CON(R'')_2$ mit R″ = je Methyl oder Äthyl sind,
-R Wasserstoff, R′ Methyl und Y -CN, $-COOCH_3$, $-COOC_2H_5$, $-CON(CH_3)_2$ oder $-CON(C_2H_5)_2$ sind,
-R Wasserstoff, R′ und Y je -CN, $-COOCH_3$ oder $-COOC_2H_5$ sind,
-R′ Wasserstoff, R Methyl und Y -CN, $-COOCH_3$, $-COOC_2H_5$, $-CON(CH_3)_2$ oder $-CON(C_2H_5)_2$ sind,
-R′ Wasserstoff, R $-CH_2COOCH_3$ und Y $-COOCH_3$ sind, oder
-R′ Wasserstoff, R $-CH_2CH_2CN$ und Y -CN sind.

Ganz besonders bevorzugt verwendet man als Verbindung der Formel III Acrylsäureäthylester, Acrylnitril und N,N-Diäthylacrylamid.

Die Katalysatoren sowie die Verbindungen der Formeln II und III sind bekannt oder können nach an sich bekannten Methoden hergestellt werden. Bezüglich der Herstellung von Verbindungen der Formel II vgl. z.B. «Organikum», 387-388, VEB Deutscher Verlag der Wissenschaften, Berlin 1964 und «Survey of Organic Syntheses», Wiley Interscience (1970), S. 860-873.

Die Verbindungen der Formeln II und III werden in mindestens stöchiometrischer Menge eingesetzt. Bevorzugt verwendet man einen Überschuss an Verbindungen der Formel III, z.B. bis ca. 1,5 Mol Verbindung der Formel III pro Säurehalogenidgruppe.

Als definitionsgemässe Palladiumverbindungen können - neben Palladium-Metall - z.B. Verbindungen der Formel IV

$M^y[PdL_n]^x$ (IV)

eingesetzt werden, worin n eine ganze Zahl von 2 bis 4,

x $2^+$ bis $2^-$,
y = −(x),

M, bei x ungleich 0, ein Gegenion und die L gleiche oder verschiedene phosphorfreie Liganden darstellen, wie z.B. Cl, Br, J, -CN, $-NO_3$, $-C_{1-12}$-Alkyl-COO, $CH_3COCHCOCH_3$, $NH_3$, 2,2'-Bipyridyl, o-Phenanthrolin, $OS(CH_3)_2$ oder -NC-Phenyl. Geeignete Verbindungen der Formel IV sind beispielsweise $PdCl_2$, $PdBr_2$, $Pd(CN)_2$, $Pd(NO_3)_2$, $Pd(O_2C-C_{1-12}\text{-Alkyl})_2$, besonders $Pd(OOCCH_3)_2$, $Pd(CH_3COCHCOCH_3)_2$, $[Pd(NH_3)_4]Cl_2$, $[PdCl_4]Na_2$, $Pd(OOCCH_3)_2$(2,2'-Bipyridyl), $Pd(OOCCH_3)_2$(o-Phenanthrolin), $PdCl_2[OS(CH_3)_2]_2$ und $PdCl_2$(NC-Phenyl)$_2$.

Ausser den oben genannten Verbindungen können auch Palladiumverbindungen anderer Oxidationsstufen eingesetzt werden, z.B. Bis-(Dibenzylidenaceton)Palladium(O) und Bis-(Isonitril)Palladium(O)-Verbindungen. Als Beispiele solcher Isonitrile seien genannt: Bis(Cyclohexylisonitril)Palladium(O), Bis-(Isopropylisonitril)Palladium(O), Bis-(tert.-Butylisonitril)Palladium(O), Bis-(p-Tolylisonitril)Palladium(O), Bis-(Phenylisonitril)Palladium(O) und Bis-(p-Methoxyphenylisonitril)Palladium(O). Davon sind Bis-Dibenzylidenaceton)Palladium(O), Bis-(Cyclohexylisonitril)Palladium(O) und Bis-(Isopropylisonitril)Palladium(O) bevorzugt.

Bevorzugt verwendet man als Katalysatoren $PdCl_2$, $PdBr_2$, $Pd(OOCCH_3)_2$, $Pd(CH_3COCHCOCH_3)_2$, $Pd(OOCCH_3)_2$(2,2'-Bipyridyl), $PdCl_2$(NC-Phenyl)$_2$, Bis-(Dibenzylidenaceton)Palladium(O) und Bis-(Cyclohexylisonitril)Palladium(O). Ganz besonders bevorzugt sind $PdCl_2$, Palladiumacetat und Bis-(Dibenzylidenaceton)Palladium(O).

Die Katalysatoren werden im allgemeinen in einer Menge von 0,0001 bis 20 Mol%, bevorzugt 0,001 bis 3 Mol%, bezogen auf die Verbindung der Formel II, eingesetzt.

Als Basen können im erfindungsgemässen Verfahren sowohl anorganische als auch organische Verbindungen, die im Reaktionsmedium ausreichend löslich sind, verwendet werden. Geeignete Basen sind beispielsweise Verbindungen der Formeln V bis VII

$(Z')^{n\oplus}(\ominus OOCQ')_{n_1}$ , (V)

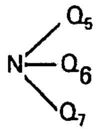 (VI) oder 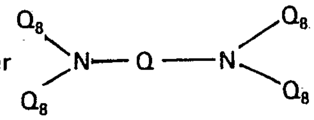 (VII)

sowie cyclische tertiäre Amine, z.B. N-Methyl- oder N-Äthylpiperidin, 1,2,2,6,6-Pentamethylpiperidin, 4-Oxo-1,2,2,6,6-pentamethylpiperidin, 1,4-Diazabicyclo[2.2.2]octan (DABCO), N-Alkylmorpholine und N-Alkylpyrrolidine, wie N-Methyl- und N-Äthylmorpholin, N-Methyl- und N-Äthylpyrrolidin, oder N,N'-Dialkylpiperazine, wie N,N'-Dimethylpiperazin.

In den obigen Formeln bedeuten:

$n_1$ die Zahl 1 oder 2,
Q' Phenyl oder $C_{1-17}$-Alkyl,
Z' ein Alkalimetallkation, ein Erdalkalimetallkation oder

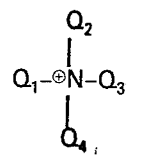 ,

Q geradkettiges oder verzweigtes Alkylen mit 2-6 C-Atomen,
$Q_1$ Wasserstoff, $C_{1-12}$-Alkyl, Cyclopentyl, Cyclohexyl, Benzyl oder Phenyl,
$Q_2$, $Q_3$ und $Q_4$ gleiches oder verschiedenes $C_{1-12}$-Alkyl,
$Q_5$ $C_{1-12}$-Alkyl, Cyclopentyl, Cyclohexyl, Phenyl oder Benzyl, das auch substituiert sein kann, beispielsweise durch ein Halogenatom, wie Chlor oder Brom, eine Alkyl- oder Alkoxygruppe mit je 1-4 und besonders 1 oder 2 C-Atomen,
$Q_6$ und $Q_7$ gleiches oder verschiedenes $C_{1-12}$-Alkyl und
$Q_8$ Methyl oder Äthyl.

Z' in der Bedeutung eines Alkalimetallkations ist besonders das Natrium- und vor allem das Lithiumkation. Alkylgruppen Q' und $Q_1$ bis $Q_7$ können geradkettig oder verzweigt sein. Stellen $Q_5$ bis $Q_7$ Alkylgruppen dar, so weisen diese mit Vorteil zusammen mindestens 9 C-Atome auf, während Alkylgruppen $Q_1$ bis $Q_4$ bevorzugt je 1-4 C-Atome aufweisen.

Beispiele von Verbindungen der Formeln V bis VII sind: das Lithiumacetat, -butyrat und -stearat, das Barium- und Calciumacetat, das Kalium-, Calcium- und Natriumstearat, das Lithium- und Natriumbenzoat, sowie die entsprechenden Trimethyl-, Tetramethyl-, Tetraäthyl- und Tetra-n-butylammoniumsalze; Triäthylamin, Tri-n-butylamin, Tri-(2-äthylhexylamin), Tri-n-octylamin und Tri-n-dodecylamin; N-Benzyldialkylamine, wie N-Benzyldimethylamin, N-benzyldiäthylamin, N-(4-Chlorbenzyl)-dimethylamin und N-(3-Methyl- oder 3-Methoxybenzyl)-dimethylamin;

N,N,N',N'-Tetramethyl- und N,N,N'N'-Tetraäthyl-äthylendiamin, N,N,N',N'-Tetramethyl-1,3-diaminopropan und N,N,N',N'-Tetramethyl-1,6-diaminohexan.

Bevorzugt verwendet man als Basen tertiäre Amine der vorerwähnten Art, vor allem N-Äthylmorpholin oder Verbindungen der Formel IV, worin $Q_5$ 4-Chlorbenzyl, 3-Methyl- oder 3-Methoxybenzyl und insbesondere Benzyl und $Q_6$ und $Q_7$ je Alkyl mit 1–4 C-Atomen, vor allem 1 oder 2 C-Atomen, darstellen oder worin $Q_5$, $Q_6$ und $Q_7$ je Alkyl mit 3–12 C-Atomen darstellen. Besonders bevorzugt sind N-Benzyldimethylamin, N-Äthylmorpholin und Tri-n-butylamin.

Die Reaktionstemperaturen für die erfindungsgemässe Umsetzung liegen zweckmässig zwischen 0 und 200°C, vorzugsweise zwischen 90 und 150°C. Sind die Säurehalogenide der Formel II flüssig, so kann die Umsetzung ohne Zusatz eines Lösungsmittels durchgeführt werden. Bevorzugt wird die Reaktion jedoch in einem gegenüber den Reaktionspartnern inerten organischen Lösungsmittel durchgeführt. Geeignete inerte organische Lösungsmittel sind z.B. gegebenenfalls chlorierte aliphatische, cycloaliphatische oder aromatische Kohlenwasserstoffe, wie n-Pentan, n-Heptan, n-Octan, Cyclopentan, Cyclohexan, Benzol, Toluol, Xylole und Chlorbenzol; aromatische, aliphatische und cyclische Äther, wie Anisol, Diäthyläther, Di-isopropyläther, Tetrahydrofuran und Dioxan; Nitrile, besonders Benzonitril und Alkylnitrile mit 2–5 C-Atomen, wie Acetonitril, Propionitril und Butyronitril; 3-Methoxypropionitril und 3-Äthoxypropionitril; N,N-Dialkylamide von aliphatischen Monocarbonsäuren mit 1–3 C-Atomen im Säureteil, wie N,N-Dimethylformamid, N,N-Dimethylacetamid und N,N-Diäthylacetamid; tertiäre Alkohole mit 4–8 C-Atomen, vor allem tert.-Butanol; aliphatische und cycloaliphatische Ketone, wie Aceton, Diäthylketon, Methylisopropylketon, Cyclopentanon und Cyclohexanon; Ester, wie Ester der Kohlensäure, z.B. Diäthylcarbonat, und Alkyl- oder Alkoxyalkylester von aliphatischen Monocarbonsäuren mit insgesamt 2–8 C-Atomen, wie Essigsäuremethyl-, -äthyl-, -n-butyl- und -isobutylester, Buttersäureäthyl- und -n-butylester, sowie 1-Acetoxy-2-methoxyäthan. Bevorzugte Lösungsmittel sind Nitrile, Ketone, Ester, cyclische Äther und aromatische Kohlenwasserstoffe der oben erwähnten Art.

Für die Umsetzung in Gegenwart anorganischer Basen eignen sich vor allem polare Lösungsmittel, wie Nitrile, Ketone und Ester. Ganz besonders bevorzugt wird die Umsetzung in Gegenwart einer organischen Base und eines aromatischen Äthers oder Kohlenwasserstoffs, vor allem Anisol, Xylolen oder Toluol, vorgenommen.

Beim erfindungsgemässen Verfahren lässt sich der Reaktionsablauf leicht anhand der CO-Entwicklung verfolgen, beispielsweise mittels eines Blasenzählers. Bei Reaktionsprodukten, die im Reaktionsgemisch beschränkt löslich sind, empfiehlt es sich, die Reaktion nach Beendigung der CO-Entwicklung zu unterbrechen und das Reaktionsprodukt direkt aufzuarbeiten.

Die erfindungsgemäss herstellbaren Verbindungen der Formel I und deren Verwendungen sind zum grossen Teil bekannt; sie können z.B. auf an sich bekannte Weise zur Herstellung von lichtvernetzbaren Polymeren verwendet werden, gegebenenfalls unter vorheriger Überführung in Derivate mit geeigneten funktionellen Gruppen, wie -COOH-, -COCl- oder Carbonsäureestergruppen, indem man sie mit Polymeren mit geeigneten funktionellen Gruppen, z.B. $NH_2$- oder OH-gruppenhaltigen Polymeren, wie Poly-(aminostyrol), Polyvinylalkohol oder Cellulose, zur Reaktion bringt. Verbindungen der Formel I, worin p die Zahl 2 bedeutet, können auch mit geeigneten Co-Componenten, besonders Diolen oder Diaminen, zu lichtvernetzbaren Polymeren kondensiert werden. Derartige Polymere finden z.B. Anwendung als sogenannte Photoresist oder für andere photographische Zwecke, wie Kopiermaterialien, Druckplatten und dergleichen [vgl. z.B. US-Patentschriften 2 670 285, 3 148 064, 3 218 168, 3 307 941, 3 387 967, 3 748 131 und 3 929 489].

Weitere Anwendungen für Zimtsäuren, Zimtsäureester und Zimtsäurenitrile sind z.B. beschrieben in Chemical Abstracts, 78, 12 699; 80, 4394, 15 756 und 29 709; 82, 17 887 und 103 009, u.a. als Klebstoffadditive, Vernetzer für Polymere, Stabilisatoren für Insektizide, Riechstoffe und Rostschutzmittel.

Verbindungen 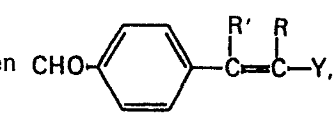

worin R und R' Wasserstoff oder $C_{1-4}$-Alkyl bedeuten, mit der Massgabe, dass eines davon Wasserstoff ist und Y die unter Formel I angegebene Bedeutung hat, und wobei der Benzolring auch weiter substituiert sein kann, z.B. durch Alkyl-, Nitril- oder Alkylsulfonylgruppen, sind auch wertvolle Zwischenprodukte zur Herstellung von optischen Aufhellern (vgl. z.B. britische Patentschrift 1 536 480).

Beispiel 1:

Zimtsäureäthylester

3,51 g (0,025 Mol) Benzoylchlorid werden zusammen mit 3,125 g (0,03125 Mol) Acrylsäureäthylester, 5,79 g (0,03125 Mol) Tri-n-butylamin, 50 ml Toluol und 0,0561 g (0,00025 Mol) Palladiumacetat in einen 100 ml Glaskolben eingefüllt und unter Rühren auf 100°C erwärmt. Es ist eine leichte Gasentwicklung zu beobachten. Nach einer Rührzeit von 4 Stunden wird abgekühlt und der Kolbeninhalt wird zweimal mit je 25 ml 2-normaler Salzsäure ausgeschüttelt. Die Toluolphase wird mit Magnesiumsulfat getrocknet und destilliert. Nach dem Abdestillieren des Toluols erhält man 2,9 g (0,0165 Mol) Zimtsäureäthylester, entsprechend einer Ausbeute von 66% d.Th.; Sdp. = 135–138°C/17·$10^2$ Pa; $n_D^{20}$=1,5592.

Beispiele 2–9:

Es wird wie in Beispiel 1 beschrieben vorgegangen, jedoch unter Verwendung von a) 50 ml Dioxan, b) 50 ml Buttersäureäthylester, c) 50 ml Propionitril, d) 50 ml Diäthylcarbonat, e) 50 ml Chlorbenzol, f) 50 ml Cyclohexan, g) 50 ml tert.-Butanol oder h) 50 ml N,N-Dimethylformamid anstelle von 50 ml Toluol. Nach der Aufarbeitung, wie in Beispiel 1 beschrieben, erhält man den Zimtsäureäthylester in vergleichbar guten Ausbeuten.

Beispiele 10–18:

Es wird wie in Beispiel 1 beschrieben vorgegangen, jedoch unter Verwendung von jeweils 0,03125 Mol der folgenden Basen: Tri-n-octylamin, Tris-(2-äthylhexyl)-amin, Äthyl-dicyclohexylamin, Triäthylamin, Äthyl-diisopropylamin, N-Methyl-2,2,6,6-tetramethylpiperidin, Lithiumacetat und Trimethyl-benzylammoniumacetat anstelle von 0,03125 Mol Tri-n-butylamin. Nach der Aufarbeitung, wie in Beispiel 1 beschrieben, erhält man den Zimtsäureäthylester in vergleichbar guten Ausbeuten.

Beispiele 19–24:

Es wird wie in Beispiel 1 beschrieben vorgegangen, jedoch unter Verwendung von jeweils 0,00025 Mol der folgenden Palladiumsverbindungen: Bis-(Dibenzylidenaceton)Palladium(O), Palladiumnitrat · $2H_2O$, Bis-(Acetylacetonato)Palladium(II), Palladiumchlorid, Palladiumcyanid anstelle von 0,00025 Mol Palladiumacetat. Nach der Aufarbeitung, wie in Beispiel 1 beschrieben, erhält man den Zimtsäureäthylester in Ausbeuten von 73–82% d.Th.

Beispiel 25:

Zimtsäure-tert.-butylester.

Es wird wie in Beispiel 1 beschrieben vorgegangen, jedoch unter Verwendung von 4 g (0,03125 Mol) Acrylsäure-tert.-butylester anstelle von 3,125 g (0,03125 Mol) Acrylsäureäthylester. Nach einer Reaktionszeit von 15 Minuten bei 133°C erhält man 2,28 g (0,0179 Mol) Zimsäure-tert.-butylester, entsprechend einer Ausbeute von 57% d.Th.; Sdp. = 144°C/10·$10^2$ Pa.

Beispiel 26:

Es wird wie in Beispiel 1 beschrieben vorgegangen, jedoch unter Verwendung von 0,0112 g (0,00005 Mol) Palladiumacetat anstelle von 0,0561 g (0,00025 Mol) Palladiumacetat. Nach einer Reaktionszeit von 6 Stunden bei 120°C erhält man den Zimtsäureäthylester in einer Ausbeute von 69% d.Th.

Beispiel 27:

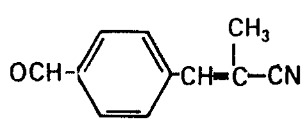

Es wird wie in Beispiel 1 beschrieben vorgegangen, jedoch unter Verwendung von 3,49 g (0,025 Mol) p-Formylbenzoylchlorid und 2,09 g (0,03125 Mol) Methacrylnitril. Nach einer Reaktionszeit von 2 Stunden bei 120°C in 50 ml p-Xylol als Lösungsmittel erhält man 1,35 g (0,0079 Mol) der obigen Verbindung als ein 60:40 Z/E-Gemisch, entsprechend einer Ausbeute von 31,6% d.Th.; Fp. = 63–64°C.

Analyse für $C_{11}H_9NO$ (Molgewicht 171):

| | | | |
|---|---|---|---|
| Ber. | C 77,17% | H 5,30% | N 8,18% |
| Gef. | C 77,11% | H 5,36% | N 8,16% |

Beispiel 27:

Zimtsäurenitril

Es wird wie in Beispiel 1 beschrieben vorgegangen, jedoch unter Verwendung von 3,31 g (0,0625 Mol) Acrylnitril, 7,02 g (0,05 Mol) Benzoylchlorid, 11,6 g (0,0625 Mol) Tri-n-butylamin, 100 ml p-Xylol und 0,1122 g (0,0005 Mol) Palladiumacetat.Nach einerReaktionszeit von 2 Stunden bei 120°C erhält man 3,47 g (0,27 Mol) Zimtsäurenitril, entsprechend einer Ausbeute von 54% d.Th. Sdp. = 134–136°C/17·$10^2$ Pa.

Beispiel 29:

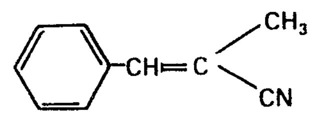

Es wird wie in Beispiel 28 beschrieben vorgegangen, jedoch unter Verwendung von 4,2 g (0,0625 Mol) Methacrylnitril anstelle von 3,31 g (0,0625 Mol) Acrylnitril. Nach einer Reaktionszeit von 3 Stunden bei 120°C erhält man 3,19 g (0,0224 Mol) der obigen Verbindung als 50:50 E/Z-Gemisch, entsprechend einer Ausbeute von 44,8% d.Th.: Sdp. = 120°C/19·$10^2$ Pa.

Beispiel 30:

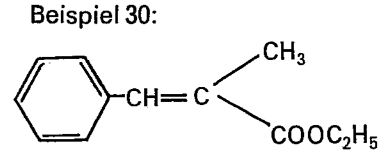

Es wird wie in Beispiel 28 beschrieben vorgegangen, jedoch unter Verwendung von 7,1 g (0,0625 Mol) Methacrylsäureäthylester anstelle von 3,31 g (0,062 Mol) Acrylnitril. Nach einer Reaktionszeit von 3 Stunden bei 120°C erhält man 2,7 g (0,0142 Mol) der obigen Verbindung als reines E-Isomeres, entsprechend einer Ausbeute von 28,4% d.Th.; Sdp. = 155–160°C/40·$10^2$ Pa.

Beispiel 31:

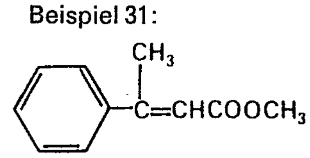

Es wird wie in Beispiel 28 beschrieben vorgegangen, jedoch unter Verwendung von 6,25 g (0,0625 Mol) Crotonsäuremethylester anstelle von 3,31 g (0,0625 Mol) Acrylnitril. Nach einer

Reaktionszeit von 6 Stunden bei 120°C erhält man 2,9 g (0,0165 Mol) der obigen Verbindung in Form von weissen Kristallen (nur E-Isomeres), entsprechend einer Ausbeute von 33% d.Th.; Fp. = 40°C.

Analyse für $C_{11}H_{12}O_2$ (Molgewicht 176):

Ber. C 74,95% H 6,78%
Gef. C 74,93% H 6,94%

Beispiel 32:
Zimtsäure-N,N-diäthylamid.
Es wird wie in Beispiel 28 beschrieben vorgegangen, jedoch unter Verwendung von 7,9 g (0,0625 Mol) Acrylsäure-N,N-diäthylamid anstelle von 3,31 g (0,0625 Mol) Acrylnitril. Nach einer Reaktionszeit von 2 Stunden bei 120°C erhält man 7,1 g (0,035 Mol) Zimtsäure-N,N-diäthylamid in Form von weissen Kristallen, entsprechend einer Ausbeute von 70% d.Th.; Fp. — 65°C.

Analyse für $C_{13}H_{17}NO$ (Molgewicht 203):

Ber. C 76,81% H 8,43% N 6,89%
Gef. C 76,63% H 8,70% N 6,89%

Beispiel 33:

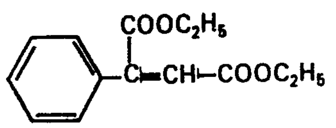

Es wird wie in Beispiel 28 beschrieben vorgegangen, jedoch unter Verwendung von 10,7 g (0,0625 Mol) Maleinsäurediäthylester anstelle von 3,31 g (0,0625 Mol) Acrylnitril. Nach einer Reaktionszeit von 4 Stunden bei 120°C erhält man 5,5 g (0,222 Mol) Phenylmaleinsäureäthylester als farblose Flüssigkeit (nur E-Isomeres), entsprechend einer Ausbeute von 44,4% d.Th.; Sdp. 123–124°C/106 Pa.

Analyse für $C_{14}H_{16}O_4$ (Molgewicht 248):

Ber. C 67,73% H 6,50%
Gef. C 68,03% H 6,72%

Beispiel 34:
Es wird wie in Beispiel 1 beschrieben vorgegangen, jedoch unter Verwendung von 4,62 g (0,025 Mol) Benzoylbromid anstelle von 3,51 g (0,025 Mol) Benzoylchlorid. Nach einer Reaktionszeit von 5 Stunden bei 120°C erhält man 0,7 g (0,004 Mol) Zimtsäureäthylester, entsprechend einer Ausbeute von 18% d.Th.

Beispiel 35:
Es wird wie in Beispiel 1 beschrieben vorgegangen, jedoch unter Verwendung von 5,8 g (0,025 Mol) Benzoyljodid anstelle von 3,51 g (0,025 Mol) Benzoylchlorid. Nach einer Reaktionszeit von 4 Stunden bei 120°C erhält man 2,78 g (0,158 Mol) Zimtsäureäthylester, entsprechend einer Ausbeute von 63% d.Th.

Beispiel 36:
4-Bromzimtsäureäthylester
Es wird wie in Beispiel 1 beschrieben vorgegangen, jedoch unter Verwendung von 5,49 g (0,025 Mol) 4-Brombenzoylchlorid anstelle von 3,51 g (0,025 Mol) Benzoylchlorid. Nach einer Reaktionszeit von 1 Stunde bei 120°C in 50 ml p-Xylol als Lösungsmittel erhält man 3,8 g (0,0149 Mol) 4-Bromzimtsäureäthylester als farblose Flüssigkeit, entsprechend einer Ausbeute von 60% d.Th.; Sdp. 180°C/21 · $10^2$ Pa.

Beispiel 37:
4-Chlorzimtsäureäthylester.
Es wird wie in Beispiel 28 beschrieben vorgegangen, jedoch unter Verwendung von 8,75 g (0,05 Mol) 4-Chlorbenzoylchlorid anstelle von 7,02 g (0,05 Mol) Benzoylchlorid. Nach der Aufarbeitung erhält man 8,3 g (0,0394 Mol) 4-Chlorzimtsäureäthylester als farblose Flüssigkeit, entsprechend einer Ausbeute von 79% d.Th.; Sdp. 108°C/106 Pa.

Analyse für $C_{11}H_{11}O_2Cl$ (Molgewicht 210,5):

Ber. C 62,72% H 5,26%
Gef. C 62,69% H 5,38%

Beispiel 38:

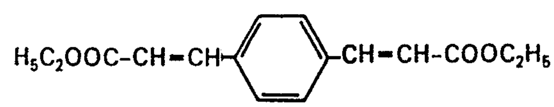

Es wird wie in Beispiel 1 beschrieben vorgegangen, jedoch unter Verwendung von 2,54 g (0,0125 Mol) Terephthalsäuredichlorid anstelle von 3,51 g (0,025 Mol) Benzoylchlorid. Nach einer Reaktionszeit von 5,5 Stunden bei 110°C erhält man 0,15 g (0,00055 Mol) Phenylen-4,4'-bisacrylsäurediäthylester, entsprechend einer Ausbeute von 4,4% d.Th.; Fp. 93–96°C.

Beispiel 39:
4-Formylzimtsäureäthylester.
Es wird wie in Beispiel 28 beschrieben vorgegangen, jedoch unter Verwendung von 8,23 g (0,05 Mol) 4-Formylbenzoylchlorid und 6,25 g (0,0625 Mol) Acrylsäureäthylester. Nach einer Reaktionszeit von 13 Stunden bei 120°C erhält man 4,7 g (0,023 Mol) 4-Formylzimtsäureäthylester, entsprechend einer Ausbeute von 46% d.Th.; Sdp. 137–140°C/13 Pa.

Beispiel 40:

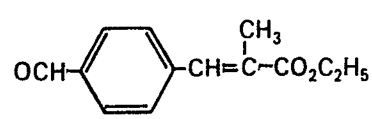

Es wird wie in Beispiel 28 beschrieben vorgegangen, jedoch unter Verwendung von 8,23 g (0,05 Mol) p-Formylbenzoylchlorid und 7,13 g

(0,0625 Mol) Methacrylsäureäthylester. Nach einer Reaktionszeit von 2 Stunden bei 120°C erhält man 3,2 g (0,0147 Mol) p-Formyl-α-methylzimtsäureäthylester, entsprechend einer Ausbeute von 29,4% d.Th.; Sdp. 127–131°C/7 Pa.

Beispiel 41:
4-Formylzimtsäurenitril.
Es wird wie in Beispiel 28 beschrieben vorgegangen, jedoch unter Verwendung von 8,23 g (0,05 Mol) 4-Formylbenzoylchlorid und 3,32 g (0,0625 Mol) Acrylnitril. Nach einer Reaktionszeit von 3 Stunden bei 120°C erhält man 3,5 g (0,0223 Mol) 4-Formylzimtsäurenitril, entsprechend einer Ausbeute von 44,6% d.Th.; Fp. 122°C.

Analyse für $C_{10}H_7NO$ (Molgewicht 157):

| | | | |
|---|---|---|---|
| Ber. | C 76,22% | H 4,49% | N 8,91% |
| Gef. | C 76,30% | H 4,55% | N 8,96% |

Beispiel 42:
3-Methoxyzimtsäurenitril.
Es wird wie in Beispiel 1 beschrieben vorgegangen, jedoch unter Verwendung von 17,05 g (0,1 Mol) 3-Methoxybenzoylchlorid, 6,63 g (0,125 Mol) Acrylnitril, 12,96 g (0,1 Mol) Äthyl-diisopropylamin, 0,1773 g (0,001 Mol) Palladiumchlorid und 50 ml Cyclohexanon als Lösungsmittel. Nach einer Reaktionszeit von 13 Stunden bei 120°C erhält man 1 g (0,0063 Mol) 3-Methoxyzimtsäurenitril, entsprechend einer Ausbeute von 6,3% d.Th.; Sdp. 159–166°C/19·$10^2$ Pa.

Beispiel 43:

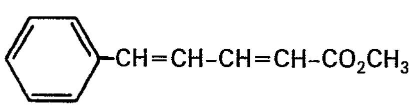

Es wird wie in Beispiel 28 beschrieben vorgegangen, jedoch unter Verwendung von 8,35 g (0,05 Mol) Zimtsäurechlorid und 5,4 g (0,0625 Mol) Acrylsäuremethylester. Nach einer Reaktionszeit von 50 Stunden bei 120°C erhält man 6,10 g (0,0325 Mol) 5-Phenyl-2,4-pentadiencarbonsäuremethylester, entsprechend einer Ausbeute von 65% d.Th.; Fp. 71°C.

Beispiel 44:
β-(2-Naphthyl)-acrylsäureäthylester.
Es wird wie in Beispiel 28 beschrieben vorgegangen, jedoch unter Verwendung von 9,55 g (0,05 Mol) 2-Naphthoylchlorid und 6,25 g (0,0625 Mol) Acrylsäureäthylester. Nach einer Reaktionszeit von 2 Stunden bei 120°C erhält man 9,9 g (0,044 Mol) β-(2-Naphthyl)-acrylsäureäthylester, entsprechend einer Ausbeute von 88% d.Th.; Sdp. 106–110°C/4 Pa.

Analyse für $C_{15}H_{14}O_2$ (Molgewicht 188):

| | | | |
|---|---|---|---|
| Ber. | C 79,62% | H 6,24% | O 14,14% |
| Gef. | C 79,39% | H 6,49% | O 14,08% |

Beispiel 45:
4-Fluorzimtsäurenitril.
Es wird wie in Beispiel 1 beschrieben vorgegangen, jedoch unter Verwendung von 15,86 g (0,1 Mol) 4-Fluorbenzoylchlorid, 26,95 g (0,1 Mol) Tri-n-hexylamin, 6,53 g (0,125 Mol) Acrylnitril und 0,575 g (0,001 Mol) Bis(Dibenzylidenaceton)Palladium(O) in 50 ml Dioxan. Nach einer Reaktionszeit von 33 Stunden bei 100°C erhält man 2,63 g (0,0179 Mol) 4-Fluorzimtsäurenitril, entsprechend einer Ausbeute von 17,9% d.Th..

Analyse für $C_9H_6FN$ (Molgewicht 147):

| | | | |
|---|---|---|---|
| Ber. | C 73,46% | H 4,11% | N 9,52% |
| Gef. | C 73,16% | H 4,19% | N 9,61% |

Beispiel 46:
3-Bromzimtsäurenitril.
Es wird wie in Beispiel 1 beschrieben vorgegangen, jedoch unter Verwendung von 21,94 g (0,1 Mol) 3-Brombenzoylchlorid, 6,63 g (0,125 Mol) Acrylnitril, 35,37 g (0,1 Mol) Tris-(2-äthylhexyl)amin und 0,3046 g (0,001 Mol) Bis-(Acetylacetonato)Palladium(II) in 50 ml Propionitril. Nach einer Reaktionszeit von 3 Stunden bei 100°C erhält man 2,05 g (0,0099 Mol) 3-Bromzimtsäurenitril, entsprechend einer Ausbeute von 9,9% d.Th.; Fp. 62°C.

Analyse für $C_9H_6NBr$ (Molgewicht 208):

| | | | |
|---|---|---|---|
| Ber. | C 51,96% | H 2,91% | N 6,73% |
| Gef. | C 52,29% | H 2,95% | N 6,91% |

Beispiel 47:
4-Nitrozimtsäureäthylester.
Es wird wie in Beispiel 1 beschrieben vorgegangen, jedoch unter Verwendung von 9,28 g (0,05 Mol) 4-Nitrobenzoylchlorid, 6,25 g (0,0625 Mol) Acrylsäureäthylester, 8,65 g (0,05 Mol) Tri-n-butylamin und 0,1122 g (0,0005 Mol) Palladiumacetat. Nach einer Reaktionszeit von 1 Stunde bei 120°C in 100 ml p-Xylol als Lösungsmittel erhält man 6,1 g (0,0276 Mol) 4-Nitrozimtsäureäthylester, entsprechend einer Ausbeute von 55,2% d.Th.; Fp. 137,5°C.

Analyse für $C_{11}H_{11}NO_4$ (Molgewicht 221):

| | | | |
|---|---|---|---|
| Ber. | C 59,73% | H 5,01% | N 6,33% |
| Gef. | C 59,47% | H 4,96% | N 6,27% |

Beispiel 48:

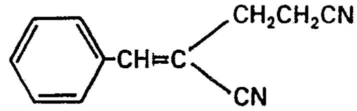

0,1122 g (5×$10^{-4}$ Mol) Palladiumacetat, 5,77 ml (50 mMol) Benzoylchlorid, 6,75 ml (62,5 mMol) 2-Methylenglutarsäuredinitril und 11,91 ml (50 mMol) Tri-n-butylamin werden in 100 ml p-Xylol während 3 Stunden bei 120°C gerührt. Das Gemisch wird mit 100 ml 2N HCl, 50 ml 2N NaOH und

50 ml Wasser ausgeschüttelt und während 15 Minuten mit 5 g Magnesiumsulfat getrocknet. Das Rohprodukt wird unter Vakuum destilliert. Man erhält 4,18 g (46% d.Th.) der obigen Verbindung als farblose Flüssigkeit.

Analyse für $C_{12}H_{10}N_2$:

Ber. C 79,10% H 5,53% N 15,38%
Gef. C 78,90% H 5,87% N 15,38%

Auf analoge Weise wie in Beispiel 48 beschrieben werden die folgenden Verbindungen hergestellt:

Beispiel 49:

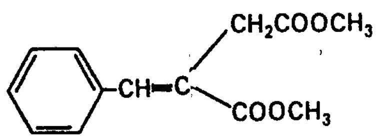

unter Verwendung von 7,9 g (50 mMol) Itaconsäuredimethylester und 6,76 g (50 mMol) N-Benzyldimethylamin; während 1,5 Stunden bei 130°C gerührt. Das Rohprodukt wird bei 105-106°C/6,7 Pa destilliert. Ausbeute 8,43 g (72% d.Th.), als hellgelbe Flüssigkeit.

Beispiel 50:

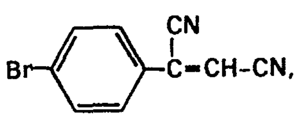

unter Verwendung von 10,97 g (50 mMol) 4-Brombenzoylchlorid, 4,88 g (62,5 mMol) Fumarsäuredinitril und 50 ml o-Xylol; während 5 Stunden bei 120°C gerührt. Das Rohprodukt wird auf Kieselgel in Toluol chromatographiert und anschliessend aus Methanol umkristallisiert. Ausbeute 1,58 g (14% d.Th.) hellbraune Kristalle vom Fp. 185,3°C.

Analyse für $C_{10}H_5BrN_2$:

Ber. C 51,54% H 2,16% N 12,02%
Gef. C 51,51% H 2,20% N 11,98%

Beispiel 51:

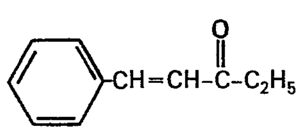 (trans),

unter Verwendung von 4,2 g (50 mMol) Äthylvinylketon und 6,76 g (50 mMol) N-Benzyldimethylamin; während 2 Stunden bei 130° gerührt. Das Rohprodukt wird im Vakuum über einer Vigreux-Kolonne destilliert und anschliessend aus n-Pentan umkristallisiert. Ausbeute 3,81 g (48% d.Th.); Fp. 35,5°C; farblose Kristalle.

Analyse für $C_{11}H_{12}O$:

Ber. C 82,46% H 7,55% O 9,99%
Gef. C 82,25% H 7,53% O 10,30%

Beispiel 52:
4-Methylzimtsäureäthylester (trans),
0,1122 g ($5 \times 10^{-4}$ Mol) Palladiumacetat, 6,61 ml (50 mMol) p-Tolylchlorid, 6,78 ml (62,5 mMol) Acrylsäureäthylester und 11,91 ml (50 mMol) Tri-n-butylamin werden in 100 ml p-Xylol während 3 Stunden bei 120°C gerührt. Das Gemisch wird mit 50 ml 2N HCl, 25 ml 2N NaOH und 25 ml Wasser ausgeschüttelt und 15 Minuten mit 5 g Magnesiumsulfat getrocknet. Das Rohprodukt wird im Vakuum destilliert. Man erhält 6,08 g (64% d.Th.) einer farblosen Flüssigkeit.

Analyse für $C_{12}H_{14}O_2$:

Ber. C 75,77% H 7,52%
Gef. C 75,73% H 6,54%

Auf analoge Weise wie in Beispiel 52 beschrieben werden die folgenden Verbindungen hergestellt:

Beispiel 53:
4-Cyanzimtsäureäthylester (trans),
unter Verwendung von 8,28 g (50 mMol) 4-Cyanbenzoylchlorid; während 2 Stunden bei 120°C gerührt. Das Rohprodukt wird einmal aus Cyclohexan und einmal aus n-Hexan umkritstallisiert. Man erhält 8,79 g (88% d.Th.) weisser Kristalle; Fp. 71°C.

Analyse für $C_{12}H_{11}NO_2$:

Ber. C 71,63% H 5,5% N 6,96%
Gef. C 71,25% H 5,38% N 6,87%

Beispiel 54:
4-Chlormethylzimtsäureäthylester (trans),
unter Verwendung von 9,45 g (50 mMol) 4-Chlormethylbenzylchlorid; während 1¼ Stunden bei 120°C gerührt. Das Rohprodukt wird aus Kieselgel in Methylenchlorid chromatographiert und anschliessend destilliert. Man erhält 4,5 g (40% d.Th.) einer farblosen Flüssigkeit.

Analyse für $C_{12}H_{13}O_2Cl$:

Ber. C 64,15% H 5,38% O 14,24% Cl 15,78%
Gef. C 64,05% H 5,87% O 14,11% Cl 15,63%

Beispiel 55:
3-Methylzimtsäureäthylester (trans),
unter Verwendung von 7,33 ml (50 mMol) m-Toluylchlorid; während 2 Std. bei 120°C gerührt. Das Rohprodukt wird im Vakuum destilliert. Man erhält 6,85 g (72% d.Th.,) einer farblosen Flüssigkeit.

Analyse für $C_{12}H_{14}O_2$:

Ber. C 75,77% H 7,42% O 16,82%
Gef. C 75,61% H 7,72% O 17,06%

Beispiel 56:
2-Chlorzimtsäureäthylester (trans),
unter Verwendung von 6,37 ml (50 mMol) 2-Chlorbenzoylchlorid; während 1,5 Std. bei 120° C gerührt. Das Rohprodukt wird im Vakuum destilliert. Man erhält 7,8 g (74% d.Th.) einer farblosen Flüssigkeit.

Analyse für $C_{11}H_{11}O_2Cl$:

Ber. C 62,77% H 5,26% O 15,19% Cl 16,83%
Gef. C 62,52% H 5,36% O 15,39% Cl 16,67%

Beispiel 57:
2-Methylzimtsäureäthylester (trans),
unter Verwendung von 6,59 ml (50 mMol) o-Toluoylchlorid; während 1,5 Std. bei 120°C gerührt. Das Rohprodukt wird im Vakuum destilliert. Man erhält 7,37 g (78% d.Th.) einer farblosen Flüssigkeit.

Analyse für $C_{12}H_{14}O_2$:

Ber. C 75,77% H 7,42% O 16,82%
Gef. C 75,60% H 7,65% O 16,92%

Beispiel 58:
3-Chlorzimtsäureäthylester (trans),
unter Verwendung von 6,42 ml (50 mMol) 3-Chlorbenzoylchlorid; während 2 Std. bei 120°C gerührt. Das Rohprodukt wird im Vakuum destilliert. Man erhält 7,87 g (75% d.Th.) einer farblosen Flüssigkeit.

Analyse für $C_{11}H_{11}O_2Cl$:

Ber. C 62,68% H 5,27% O 15,19% Cl 16,86%
Gef. C 62,63% H 5,29% O 15,27% Cl 17,34%

Beispiel 59:
3-Jodzimtsäureäthylester (trans),
unter Verwendung von 13,33 g (50 mMol) 3-Jodbenzoylchlorid; während 65 Minuten bei 120°C gerührt. Das Rohprodukt wird im Vakuum destilliert und anschliessend aus Methanol/Wasser umkristallisiert. Man erhält 4,7 g (31% d.Th.) weisser Kristalle; Fp. 36,3%.

Analyse für $C_{11}H_{11}O_2J$:

Ber. C 43,71% H 3,68% O 10,60% J 42,02%
Gef. C 44,07% H 3,61% O 10,63% J 41,25%

Beispiel 60:
2-Acetoxyzimtsäureäthylester (trans),
unter Verwendung von 9,93 g (50 mMol) 2-Acetylsalicylsäurechlorid; während 50 Minuten bei 120°C gerührt. Das Rohprodukt wird im Vakuum destilliert. Man erhält 6,66 g (57% d.Th.) einer farblosen Flüssigkeit.

Analyse für $C_{13}H_{14}O_4$:

Ber. C 66,66% H 6,03% O 27,23%
Gef. C 66,18% H 6,30% O 27,06%

Beispiel 61:
4-Phenylzimtsäureäthylester (trans),
unter Verwendung von 10,83 g (50 mMol) Biphenyl-4-carbonsäurechlorid; während 1,5 Std. bei 120°C gerührt. Das Rohprodukt wird auf Kieselgel in Methylenchlorid chromatographiert und aus n-Hexan umkristallisiert. Man erhält 8,03 g (64% d.Th.) weisser Kristalle; Fp. 88,6°C.

Analyse für $C_{17}H_{16}O_2$:

Ber. C 80,93% H 6,39% O 12,68%
Gef. C 80,77% H 6,28% O 12,89%

Beispiel 62:
4-Methoxycarbonylzimtsäureäthylester (trans),
unter Verwendung von 9,93 g (50 mMol) Terephthalsäurechlorid-monomethylester; während 1,5 Std. bei 120°C gerührt. Das Rohprodukt wird im Vakuum destilliert. Man erhält 3,63 g (31% d.Th.) des genannten Esters.

Analyse für $C_{13}H_{14}O_4$:

Ber. C 66,66% H 6,03% O 27,32%
Gef. C 66,07% H 5,97% O 27,08%

Beispiel 63:

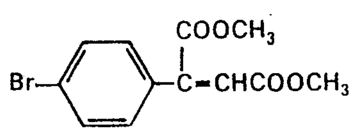
(E-Isomer),

unter Verwendung von 10,97 g (50 mMol) Brombenzoylchlorid und 9,01 g (62,5 mMol) Maleinsäuredimethylester; während 5 Std. bei 120°C gerührt. Das Rohprodukt wird im Vakuum destilliert. Man erhält 4,36 g (31% d.Th.) der obigen Verbindung (destilliert bei 122°C/13,3 Pa) als gelbe Flüssigkeit.

Analyse für $C_{12}H_{11}BrO_4$:

Ber. C 48,19% H 3,71% O 21,40%
Gef. C 48,61% H 3,90% O 21,25%

Beispiel 64:

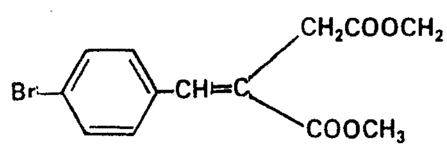
(E-Isomer)

analog Beispiel 63, unter Verwendung von 9,88 g (62,5 mMol) Itaconsäuredimethylester; während 3¼ Std. bei 120°C gerührt. Nach dem Destillieren des Rohprodukts im Vakuum erhält man 8,2 g (53% d.Th.) der obigen Verbindung als gelbe Flüssigkeit (destilliert bei 143-150°C/13,3 Pa).

Beispiel 65:

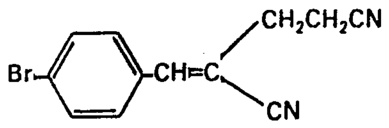

(E-Isomer)

analog Beispiel 63, unter Verwendung von 6,63 g (62,5 mMol) 2-Methylenglutarsäuredinitril; während 2¼ Std. bei 120°C gerührt. Das Rohprodukt wird im Vakuum destilliert und anschliessend aus Methanol umkristallisiert. Man erhält 3,5 g (27% d.Th.) der obigen Verbindung als weisse Kristalle; Fp. 60,2°C.

Analyse für $C_{12}H_9BrN_2$:

Ber. C 55,20% H 3,48% N 10,73%
Gef. C 55,59% H 3,58% N 10,48%

Beispiel 66:
4-Phenylzimtsäurenitril (trans),
unter Verwendung von 10,83 g (50 mMol) Biphenyl-4-carbonsäurechlorid und 3,31 g (62,5 mMol) Acrylnitirl; während 2 Std. bei 120°C gerührt. Das Rohprodukt wird im Soxhlet mit 100 ml n-Hexan extrahiert und aus Methanol umkristallisiert. Man erhält 3,83 g (37% d.Th.) gelber Kristalle; Fp. 43,7°C.

Analyse für $C_{15}H_{11}N$:

Ber. C 87,77% H 5,40% N 6,82%
Gef. C 87,64% H 5,34% N 6,76%

Beispiel 67:
4-Phenylzimtsäure-N,N-diäthylamid (trans),
unter Verwendung von 10,83 g (50 mMol) Biphenyl-4-carbonsäurechlorid und 7,94 g (62,5 mMol) N,N-Diäthylacrylamid; während 50 Minuten bei 120°C gerührt. Das Rohprodukt wird im Soxhlet mit 125 ml n-Hexan extrahiert und einmal aus Diäthyläther umkristallisiert. Man erhält 8,5 g (15% d.Th.) hellgelber Kristalle; Fp. 113,3°C.

Analyse für $C_{10}H_{21}NO$:

Ber. C 81,72% H 7,53% N 5,02%
Gef. C 81,44% H 7,50% N 4,91%

Beispiel 68:
3,4-Dimethylzimtsäurenitril.
0,448 g (2 mMol) Palladiumacetat, 33,7 g (0,2 Mol) 3,4-Dimethylbenzoylchlorid, 13,25 g (0,25 Mol) Acrylnitril und 37,06 g (0,2 Mol) Tri-n-butylamin in 200 ml p-Xylol werden während 1,5 Std. bei 120°C gerührt. Das Gemisch wird mit 200 ml 2N HCl, 200 ml 2N NaOH und 100 ml Wasser ausgeschüttelt und während 15 Minuten mit 20 g Magnesiumsulfat getrocknet. Das Rohprodukt wird auf Kieselgel in Methylenchlorid chromatographiert und aus n-Hexan umkristallisiert. Man erhält 10,0 g (32% d.Th.) 3,4-Dimethylzimtsäurenitril als hellgelbe Kristalle; Fp. 93,9°C.

Analyse für $C_{11}H_{11}N$:

Ber. C 84,04% H 7,05% N 8,91%
Gef. C 83,65% H 6,87% N 9,03%

Im wesentlichen wie in Beispiel 68 beschrieben werden die folgenden Verbindungen hergestellt:

Beispiel 69:
3,4-Dimethylzimtsäure-N,N-diäthylamid,
unter Verwendung von 31,75 g (0,25 mMol) N,N-Diäthylacrylamid; während 2 Std. bei 120°C gerührt. Das Rohprodukt wird im Vakuum destilliert und anschliessend aus n-Hexan umkristallisiert. Man erhält 13,3 g (29% d.Th.) der genannten Verbindung in Form weisser Kristalle; Fp. 36,6°C.

Analyse für $C_{15}H_{21}NO$:

Ber. C 77,88% H 9,15% N 6,06%
Gef. C 77,22% H 8,93% N 6,01%

Beispiel 70:
3,4-Dimethylzimtsäureäthylester,
unter Verwendung von 27,08 ml (0,2 Mol) Acrylsäureäthylester; während 2 Std. bei 120°C gerührt. Das Rohprodukt wird bei 125-128°C/173 Pa destilliert. Man erhält 12,72 g (62% d.Th.) einer farblosen Flüssigkeit.

Analyse für $C_{13}H_{16}O_2$:

Ber. C 76,44% H 7,90% O 15,67%
Gef. C 76,79% H 8,22% O 15,88%

Beispiel 71:

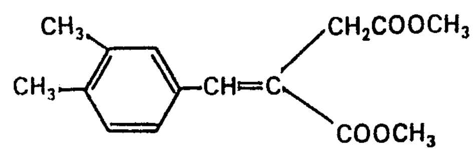

unter Verwendung von 31,6 g (0,2 Mol) Itaconsäuredimethylester und 27,04 g (0,2 Mol) N-Benzyldimethylamin; während 4 Std. bei 130°C gerührt. Das Rohprodukt wird auf Kieselgel in Methylenchlorid chromatographiert und dann im Vakuum destilliert. Man erhält 23,1 g (44% d.Th.) einer gelben Flüssigkeit.

Analyse für $C_{15}H_{18}O_4$:

Ber. C 68,69% H 6,92% O 24,40%
Gef. C 68,9% H 6,8% O 24,4%

Beispiel 72:

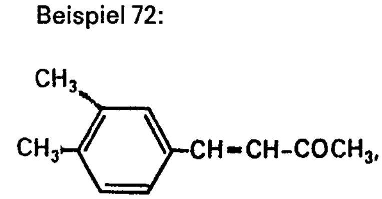

,

unter Verwendung von 14,18 g (0,2 Mol) Vinylmethylketon und 27,04 g (0,2 Mol) N-Benzyldimethylamin; 1,5 Std. bei 130°C gerührt. Das Rohprodukt wird auf Kieselgel mit Methylenchlorid chromatographiert und dann aus n-Hexan umkristallisiert. Man erhält 3,2 g (9% d.Th.) gelber Kristalle.

Beispiel 73:
3,4-Dichlorzimtsäurenitril,
unter Verwendung von 41,89 g (0,2 Mol) 3,4-Dichlorbenzoylchlorid; während 2 Std. bei 120°C gerührt. Das Rohprodukt wird auf Kieselgel in Methylenchlorid chromatographiert und dann aus n-Hexan umkristallisiert. Man erhält 7,3 g (24% d.Th.) hellgelber Kristalle; Fp. 98,2°C.

Analyse für $C_9H_5Cl_2N$:

| | | | |
|---|---|---|---|
| Ber. | C 54,58% | H 2,55% | N 7,07% |
| Gef. | C 54,5% | H 2,6% | N 7,6% |

Beispiel 74:
3,4-Dichlorzimtsäure-N,N-diäthylamid
analog Beispiel 73, unter Verwendung von 31,75 g (0,2 Mol) N,N-Diäthylacrylamid; während 50 Minuten bei 120°C gerührt. Das Rohprodukt wird im Vakuum destilliert und dann aus Diäthyläther/n-Hexan umkristallisiert. Man erhält 18,4 g (34% d.Th.) weisser Kristalle; Fp. 60,7°C.

Analyse für $C_{13}H_{15}Cl_2NO$:

| | | | | |
|---|---|---|---|---|
| Ber. | C 57,37% | H 5,56% | N 5,15% | Cl 26,05% |
| Gef. | C 57,45% | H 5,70% | N 5,11% | Cl 25,94% |

Beispiel 75:
3,4-Dichlorzimtsäureäthylester,
analog Beispiel 73, unter Verwendung von 20,02 g (0,2 Mol) Acrylsäureäthylester und 27,04 g (0,2 Mol) N-Benzyldimethylamin; während 40 Minuten bei 130°C gerührt. Das Rohprodukt wird im Soxhlet mit 300 ml n-Pentan extrahiert und dann aus Methanol umkristallisiert. Man erhält 25,4 g (52% d.Th.) weisser Kristalle; Fp. 56°C.

Analyse für $C_{11}H_{10}O_2Cl_2$:

| | | | | |
|---|---|---|---|---|
| Ber. | C 53,90% | H 4,11% | O 13,05% | Cl 28,93% |
| Gef. | C 53,77% | H 3,95% | O 13,24% | Cl 29,00% |

Beispiel 76:

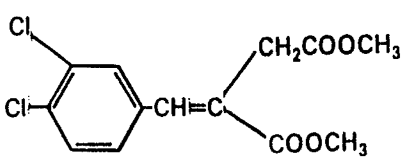

,

analog Beispiel 73 unter Verwendung von 31,6 g (0,2 Mol) Itaconsäuredimethylester und 27,04 g (0,2 Mol) N-Benzyldimethylamin; während 1,5 Stunden bei 130°C gerührt. Das Rohprodukt wird im Vakuum bei 150–158°C/77 Pa destilliert. Man erhält 28,3 g (47% d.Th.) einer gelben Flüssigkeit.

Analyse für $C_{13}H_{12}Cl_2O_4$:

| | | | |
|---|---|---|---|
| Ber. | C 51,51% | H 3,99% | O 21,11% |
| Gef. | C 51,18% | H 4,00% | O 21,27% |

Beispiel 77:

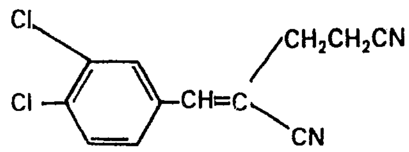

,

analog Beispiel 73, unter Verwendung von 21,2 g (0,2 Mol) 2-Methylenglutarsäuredinitril und 27,04 g (0,2 Mol) N-Benzyldimethylamin; während 2,5 Std. bei 130°C gerührt. Das Rohprodukt wird auf Kieselgel in Methylenchlorid chromatographiert und danach im Vakuum destilliert. Anschliessend wird es aus Tetrachlorkohlenstoff/Cyclohexan umkristallisiert. Man erhält 15,8 g (31% d.Th.) hellgelber Kristalle; Fp. 55,3°C.

Analyse für $C_{12}H_8Cl_2N_2$:

| | | | |
|---|---|---|---|
| Ber. | C 57,40% | H 3,12% | N 11,16% |
| Gef. | C 57,63% | H 3,37% | N 11,41% |

Beispiel 78:
2-Methyl-5-nitrozimtsäurenitril.
0,336 g (1,5 mMol) Palladiumacetat, 29,93 g (150 mMol) 2-Methyl-5-nitrobenzylchlorid, 9,94 g (187,5 mMol) Acrylnitril und 27,80 g (150 mMol) Tri-n-butylamin werden in 150 ml p-Xylol während 80 Minuten bei 130° gerührt. Dann wird wie in Beispiel 68 beschrieben ausgeschüttelt und getrocknet. Das Rohprodukt wird auf Kieselgel in Toluol chromatographiert und dann aus Tetrachlorkohlenstoff/Cyclohexan umkristallisiert. Man erhält 6,3 g (22% d.Th.) weisser Kristalle; Fp. 115,6°C.

Analyse für $C_{10}H_8N_2O_2$:

| | | | |
|---|---|---|---|
| Ber. | C 63,83% | H 4,29% | N 14,89% |
| Gef. | C 63,8% | H 4,2% | N 15,1% |

Beispiel 79:
2-Methyl-5-nitrozimtsäure-N,N-diäthylamid,
analog Beispiel 78 unter Verwendung von 23,8 g (187,5 mMol) N,N-Diäthylacrylamid; während 70 Minuten bei 120°C gerührt. Das Rohprodukt wird im Vakuum destilliert und dann aus Diäthyläther umkristallisiert. Man erhält 9,9 g (25% d.Th.) hellgelber Kristalle; Fp. 61,9°C.

Analyse für $C_{14}H_{18}N_2O_3$:

| | | | |
|---|---|---|---|
| Ber. | C 64,11% | H 6,92% | N 10,68% |
| Gef. | C 64,08% | H 7,04% | N 10,53% |

Beispiel 80:
2-Methyl-5-nitrozimtsäureäthylester,

analog Beispiel 78 unter Verwendung von 20,31 ml (187,5 mMol) Acrylsäureäthylester; während 90 Minuten bei 120°C gerührt. Das Rohprodukt wird im Soxhlet mit 270 ml Cyclohexan extrahiert, auf Kieselgel in Methylenchlorid chromatographiert und dann aus Diäthyläther/Äthanol umkristallisiert. Man erhält 9,24 g (39,3% d.Th.) hellgelber Kristalle vom Fp. 44,0°C.

Analyse für $C_{12}H_{13}NO_4$:

| | | | | |
|---|---|---|---|---|
| Ber. | C 61,27% | H 5,57% | N 5,96% | O 27,21% |
| Gef. | C 61,29% | H 5,52% | N 6,02% | O 26,96% |

Beispiel 81:

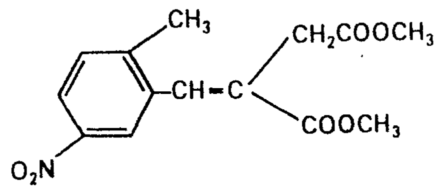

analog Beispiel 78 unter Verwendung von 24,93 g (125 mMol) Itaconsäuredimethylester und 23,17 g (125 mMol) Tri-n-butylamin; während 1 Std. bei 130°C gerührt. Das Rohprodukt wird im Vakuum destilliert. Man erhält 11,4 g (31% d.Th.) einer gelben Flüssigkeit (destilliert bei 184–188°C/93 Pa).

Analyse für $C_{14}H_{15}O_6N$:

| | | | |
|---|---|---|---|
| Ber. | C 57,34% | H 5,16% | N 4,78% |
| Gef. | C 56,99% | H 5,11% | N 4,85% |

Beispiel 82:
2-Methyl-3-nitrozimtsäurenitril,
analog Beispiel 78 unter Verwendung von 29,93 g (150 mMol) 2-Methyl-3-nitrobenzylchlorid; während 1¾ Std. bei 120°C gerührt. Das Rohprodukt wird auf Kieselgel in Chloroform chromatographiert und dann aus Tetrachlorkohlenstoff umkristallisiert. Man erhält 10,2 g (27% d.Th.) hellgelber Kristalle; Fp. 135,8°C.

Analyse für $C_{10}H_8N_2O_2$:

| | | | |
|---|---|---|---|
| Ber. | C 63,83% | H 4,29% | N 14,89% |
| Gef. | C 63,51% | H 4,15% | N 14,89% |

Beispiel 83:
2-Methyl-3-nitrozimtsäure-N,N-diäthylamid,
analog Beispiel 82 unter Verwendung von 23,81 g (187,5 mMol) N,N-Diäthylacrylamid; während 45 Minuten bei 120°C gerührt. Das Rohprodukt wird im Vakuum destilliert und aus Diäthyläther umkristallisiert. Man erhält 16,6 g (42% d.Th.) weisser Kristalle; Fp. 74,3°C.

Analyse für $C_{14}H_{18}N_2O_3$:

| | | | |
|---|---|---|---|
| Ber. | C 64,11% | H 6,92% | N 10,68% |
| Gef. | C 64,63% | H 7,04% | N 10,68% |

Beispiel 84:
2-Methyl-3-nitrozimtsäureäthylester,
analog Beispiel 82 unter Verwendung von 20,31 ml (187,5 mMol) Acrylsäureäthylester; während 1,5 Std. bei 120°C gerührt. Das Rohprodukt wird einmal aus Diäthyläther und einmal aus n-Hexan umkristallisiert. Man erhält 12,9 g (55% d.Th.) hellgelber Kristalle; Fp. 58,2°C.

Analyse für $C_{12}H_{13}NO_4$:

| | | | | |
|---|---|---|---|---|
| Ber. | C 61,27% | H 5,57% | N 5,96% | O 27,21% |
| Gef. | C 61,39% | H 5,67% | N 5,96% | O 27,33% |

Beispiel 85:

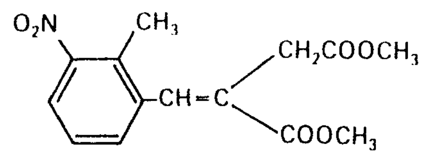

analog Beispiel 82 unter Verwendung von 23,70 g (150 mMol) Itaconsäuredimethylester und 17,25 g (150 mMol) N-Äthylmorpholin; während 1 Std. bei 130°C gerührt. Das Rohprodukt wird im Soxhlet extrahiert und aus n-Pentan umkristallisiert. Man erhält 23,4 g (53% d.Th.) hellgelber Kristalle vom Fp. 68,4°C.

Analyse für $C_{14}H_{15}NO_6$:

| | | | |
|---|---|---|---|
| Ber. | C 57,34% | H 5,16% | N 4,78% |
| Gef. | C 57,30% | H 5,32% | N 4,91% |

Beispiel 86:
3,5-Dimethoxyzimtsäurenitril,
analog Beispiel 68 unter Verwendung von 40,1 g (0,2 Mol) 3,5-Dimethoxybenzoylchlorid; während 2¼ Std. bei 120°C gerührt. Das Rohprodukt wird auf Kieselgel chromatographiert und dann aus Cyclohexan umkristallisiert. Man erhält 12,2 g (32% d.Th.) weisser Kristalle vom Fp. 125,7°C.

Analyse für $C_{11}H_{11}NO_2$:

| | | | |
|---|---|---|---|
| Ber. | C 69,83% | H 5,86% | O 7,40% |
| Gef. | C 69,6% | H 5,8% | N 7,2% |

Beispiel 87:
3,5-Dimethoxyzimtsäure-N,N-diäthylamid,
analog Beispiel 86 unter Verwendung von 31,76 g (0,25 Mol) N,N-Diäthylacrylamid; während 80 Minuten bei 120°C gerührt. Das Rohprodukt wird im Vakuum destilliert. Man erhält 10,0 g (39% d.Th.) einer gelben Flüssigkeit (destilliert bei 188–192°C/27 Pa).

Analyse für $C_{15}H_{21}NO_3$:

| | | | |
|---|---|---|---|
| Ber. | C 68,41% | H 8,04% | N 5,32% |
| Gef. | C 67,49% | H 8,30% | N 5,16% |

Beispiel 88:
3,5-Dimethoxyzimtsäureäthylester,
analog Beispiel 86 unter Verwendung von 27,08 ml (0,2 Mol) Acrylsäureäthylester; während 2 Std. bei 120°C gerührt. Das Rohprodukt wird im Vakuum destilliert. Man erhält 39,6 g (84% d.Th.) einer farblosen Flüssigkeit (destilliert bei 150–151°C/133 Pa).

Analyse für $C_{13}H_{16}O_4$:

| | | | |
|---|---|---|---|
| Ber. | C 66,09% | H 6,83% | O 27,09% |
| Gef. | C 66,21% | H 6,73% | O 27,20% |

Beispiel 89:

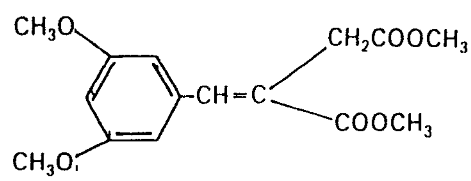

analog Beispiel 86 unter Verwendung von 31,6 g (0,2 Mol) Itaconsäuredimethylester und 27,04 g (0,2 Mol) N-Benzyldimethylamin; während 70 Minuten bei 130°C gerührt. Nach dem Destillieren des Rohproduktes erhält man 38,4 g (65% d.Th.) der obigen Verbindung als gelbe Flüssigkeit (destilliert bei 147–150°C/13 Pa).

Analyse für $C_{15}H_{20}O_6$:

| | | | |
|---|---|---|---|
| Ber. | C 60,80% | H 6,81% | O 32,40% |
| Gef. | C 61,03% | H 6,31% | O 32,82% |

Beispiel 90:

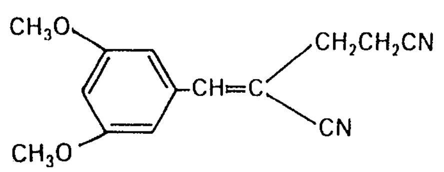

analog Beispiel 86 unter Verwendung von 21,2 g (0,2 Mol) 2-Methylenglutarsäurenitril und 27,04 g (0,2 Mol) N-Benzyldimethylamin; während 4 Std. bei 130°C gerührt. Das Rohprodukt wird im Vakuum destilliert und aus Cyclohexan umkristallisiert. Man erhält 17,1 g (35% d.Th.) der obigen Verbindung in Form weisser Kristalle; Fp. 71,6°C.

Analyse für $C_{14}H_{14}N_2O_2$:

| | | | |
|---|---|---|---|
| Ber. | C 69,41% | H 5,83% | N 11,57% |
| Gef. | C 69,72% | H 5,86% | N 11,88% |

Beispiel 91:
3,4,5-Trimethoxyzimtsäurenitril,
analog Beispiel 68 unter Verwendung von 46,13 g (0,2 Mol) 3,4,5-Trimethoxybenzoylchlorid; während 1,5 Std. bei 120°C gerührt. Das Rohprodukt wird im Vakuum destilliert und anschliessend aus Diäthyläther/n-Hexan umkristallisiert. Man erhält 14,3 g (33% d.Th.) hellgelber Kristalle vom Fp. 92,1°C.

Analyse für $C_{12}H_{13}NO_3$:

| | | | |
|---|---|---|---|
| Ber. | C 65,74% | H 5,98% | N 6,39% |
| Gef. | C 65,83% | H 6,16% | N 6,27% |

Beispiel 92:
3,4,5-Trimethoxyzimtsäure-N,N-diäthylamid,
analog Beispiel 91 unter Verwendung von 31,75 g (0,2 Mol) N,N-Diäthylacrylamid; während 2,5 Std. bei 120°C gerührt. Das Rohprodukt wird im Vakuum destilliert und anschliessend aus Diäthyläther/Dioxan umkristallisiert. Man erhält 9,4 g (16% d.Th.) hellgelber Kristalle vom Fp. 131,7°C.

Analyse für $C_{16}H_{23}NO_4$:

| | | | |
|---|---|---|---|
| Ber. | C 65,51% | H 7,90% | N 4,78% |
| Gef. | C 65,43% | H 7,73% | N 4,69% |

Beispiel 93:
3,4,5-Trimethoxyzimtsäureäthylester,
analog Beispiel 91 unter Verwendung von 27,08 ml (0,25 Mol) Acrylsäureäthylester; während 2 Std. bei 120°C gerührt. Das Rohprodukt wird im Vakuum destilliert und aus n-Hexan umkristallisiert. Man erhält 23,6 g (47% d.Th.) weisser Kristalle vom Fp. 63,4%.

Analyse für $C_{14}H_{18}O_5$:

| | | | |
|---|---|---|---|
| Ber. | C 63,15% | H 6,8% | O 30,04% |
| Gef. | C 63,40% | H 6,83% | O 30,09% |

Beispiel 94:

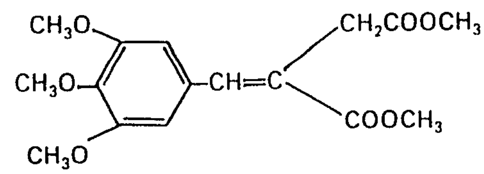

analog Beispiel 91 unter Verwendung von 31,6 g (0,2 Mol) Itaconsäuredimethylester und 27,04 g (0,2 Mol) N-Benzyldimethylamin; während 100 Minuten bei 130°C gerührt. Das Rohprodukt wird auf Kieselgel in Diäthyläther chromatographiert und dann im Vakuum destilliert. Man erhält 20,6 g (32% d.Th.) einer gelben Flüssigkeit (destilliert bei 181–184°C/67 Pa).

Analyse für $C_{16}H_{20}O_7$:

| | | | |
|---|---|---|---|
| Ber. | C 59,26% | H 6,22% | O 34,53% |
| Gef. | C 59,22% | H 6,14% | O 34,64% |

Beispiel 95:

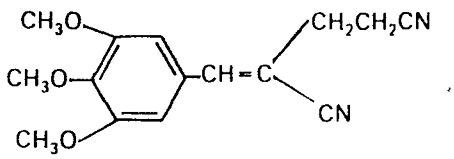

analog Beispiel 91 unter Verwendung von 21,2 g (0,2 Mol) 2-Methylenglutarsäuredinitril und 27,04 g (0,2 Mol) N-Benzyldimethylamin; während 1,5 Std. bei 130°C gerührt. Das Rohprodukt wird im Soxhlet mit Cyclohexan extrahiert und dann aus Methanol umkristallisiert. Man erhält 4,9 g (9% d.Th.) weisser Kristalle vom Fp. 141,3°C.

Analyse für $C_{15}H_{16}N_2O_3$:

| | | | |
|---|---|---|---|
| Ber. | C 66,17% | H 5,92% | N 10,29% |
| Gef. | C 65,9% | H 6,0% | N 10,2% |

Beispiel 96:
Pentafluorzimtsäureäthylester.

0,0561 g (0,25 mMol) Palladiumacetat, 5,76 g (25 mMol) Pentafluorbenzoylchlorid, 2,50 g (25 mMol) Acrylsäureäthylester und 2,88 g (25 mMol) N-Äthylmorpholin werden in 100 ml Xylol während 6,5 Std. bei 130°C gerührt. Das Rohprodukt wird im Vakuum destilliert. Man erhält 3,9 g (59% d.Th.) einer farblosen Flüssigkeit (destilliert bei 61–64°C/12 Pa).

Analyse für $C_{11}H_7F_5O_2$:

| | | | |
|---|---|---|---|
| Ber. | C 49,64% | H 2,65% | F 35,69% |
| Gef. | C 49,76% | H 2,72% | F 35,90% |

Beispiel 97:

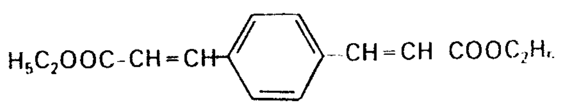

0,1122 g ($5 \times 10^{-4}$ Mol) Palladiumacetat, 5,08 g ($2,5 \times 10^{-2}$ Mol) Terephthalsäuredichlorid, 6,78 ml ($6,25 \times 10^{-2}$ Mol) Acrylsäureäthylester und 11,91 ml ($5 \times 10^{-3}$ Mol) Tri-n-butylamin in 100 ml Xylol werden 1 Std. bei 120°C gerührt. Das Ausschütteln erfolgt wie in Beispiel 48 beschrieben. Das Rohprodukt wird destilliert und einmal aus Cyclohexan umkristallisiert. Man erhält 4,08 g (60% d.Th.) weisser Kristalle; Fp. 97,1°C.

Analyse für $C_{16}H_{18}O_4$:

| | | |
|---|---|---|
| Ber. | C 70,06% | H 6,62% |
| Gef. | C 69,89% | H 6,44%. |

Auf analoge Weise wie in Beispiel 97 beschrieben werden die Verbindungen gemäss den Beispielen 98–104 hergestellt.

Beispiel 98:
1,4-Bis-(N,N-Dimethylcarbamoylvinyl)-benzol,

analog Beispiel 97 unter Verwendung von 0,896 g (4 mMol) Palladiumacetat, 40,6 g (0,2 Mol) Terephthalsäuredichlorid, 49,5 g (0,5 Mol) N,N-Dimethylacrylamid und 74,12 g (0,4 Mol) Tri-n-butylamin; während 1 Std. bei 120°C gerührt. Das Rohprodukt wird aus Dioxan umkristallisiert. Man erhält 11,6 g (21% d.Th.) gelber Kristalle vom Fp. 247,0°C.

Analyse für $C_{16}H_{20}N_2O_2$:

| | | | |
|---|---|---|---|
| Ber. | C 70,56% | H 7,40% | N 10,29% |
| Gef. | C 70,27% | H 7,34% | N 10,10% |

Beispiel 99:

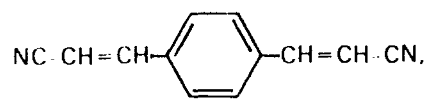

analog Beispiel 98 unter Verwendung von 26,5 g (0,5 Mol) Acrylnitril; während 3 Std. bei 120°C gerührt. Das Rohprodukt wird im Soxhlet extrahiert und aus Dioxan/Isopropanol umkristallisiert. Man erhält 6,8 g (19% d.Th.) gelber Kristalle.

Analyse für $C_{12}H_8N_2$:

| | | | |
|---|---|---|---|
| Ber. | C 79,98% | H 4,48% | N 15,55% |
| Gef. | C 79,70% | H 4,68% | N 15,50% |

Beispiel 100:
1,3-Bis-Äthoxycarbonylvinylbenzol,

analog Beispiel 98 unter Verwendung von 40,6 g (0,2 Mol) Isophthalsäuredichlorid und 50 g (0,5 Mol) Acrylsäureäthylester; während 100 Minuten bei 120°C gerührt. Das Rohprodukt wird im Vakuum destilliert und dann aus n-Hexan/Cyclohexan umkristallisiert. Man erhält 13,4 g (24% d.Th.) weisser Kristalle vom Fp. 51,5°C.

Analyse für $C_{16}H_{18}O_4$:

| | | | |
|---|---|---|---|
| Ber. | C 70,06% | H 6,62% | O 23,33% |
| Gef. | C 70,34% | H 6,61% | O 23,51% |

Beispiel 101:

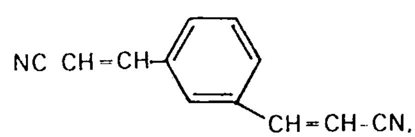

analog Beispiel 100 unter Verwendung von 26,5 g (0,5 Mol) Acrylnitril; während 3,5 Std. bei 120°C gerührt. Das Rohprodukt wird im Soxhlet extrahiert und dann aus Cyclohexan umkristallisiert. Man erhält 2,2 g (6% d.Th.) hellgelber Kristalle.

Analyse für $C_{12}H_8N_2$:

| | | | |
|---|---|---|---|
| Ber. | C 79,98% | H 4,48% | N 14,55% |
| Gef. | C 79,10% | H 4,77% | N 15,28% |

Beispiel 102:
2,6-Bis-(2-Äthoxycarbonylvinyl)-naphthalin.

0,1683 g (0,75 mMol) Palladiumacetat, 9,49 g (37,5 mMol) Naphthalin-2,6-dicarbonsäuredichlorid, 9,38 g (93,75 mMol) Acrylsäureäthylester und 13,89 g (75 mMol) Tri-n-butylamin in 150 ml p-Xylol werden während 2 Std. bei 120°C gerührt. Das Rohprodukt wird auf Kieselgel in Methylenchlorid chromatographiert und dann aus Cyclohexan umkristallisiert. Man erhält 4,11 g (34% d.Th.) gelber Kristalle vom Fp. 150–151°C.

Analyse für $C_{20}H_{20}O_4$:

| | | | |
|---|---|---|---|
| Ber. | C 74,06% | H 6,2% | O 19,73% |
| Gef. | C 73,85% | H 6,41% | O 19,56% |

Beispiel 103:

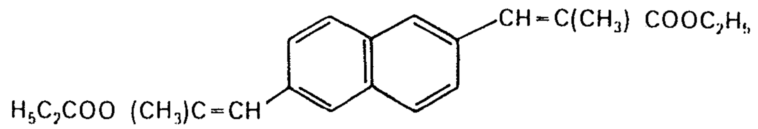

unter Verwendung von 0,0224 g (0,1 mMol) Palladiumacetat, 1,27 g (5 mMol) Naphthalin-2,6-dicarbonsäuredichlorid, 1,43 g (12,5 mMol) Methacrylsäureäthylester und 2,31 g (12,5 mMol) Tri-n-butylamin in 20 ml Toluol, die während 3,5 Std. unter Rückfluss gekocht werden. Das Rohprodukt wird im Soxhlet extrahiert und aus n-Hexan umkristallisiert. Man erhält 0,14 g (7% d.Th.) weisser Blättchen vom Fp. 107–108°C.

Beispiel 104:

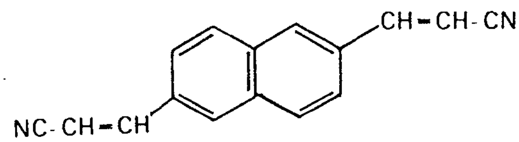

analog Beispiel 102 unter Verwendung von 4,98 g (93,75 mMol) Acrylnitril; während 2 Std. bei 120°C gerührt. Das Rohprodukt wird im Soxhlet extrahiert und dann aus Tetrahydrofuran/Methanol umkristallisiert. Man erhält 3,12 g (30% d.Th.) gelber Kristalle vom Fp. 227–229°C.

Beispiel 105:
1,4-Bis-(2-Äthoxycarbonyl-vinyl)-naphthalin.

0,0561 g (0,25 mMol) Palladiumacetat, 3,16 g (12,5 mMol) Naphthalin-1,4-dicarbonsäuredichlorid, 3,12 g (31,25 mMol) Acrylsäureäthylester und 4,63 g (25 mMol) Tri-n-butylamin in 150 ml p-Xylol werden während 2 Std. bei 120°C gerührt. Das Rohprodukt wird auf Kieselgel in Methylenchlorid chromatographiert und dann aus Cyclohexan umkristallisiert. Man erhält 2,31 g (57% d.Th.) hellgelber Kristalle vom Fp. 90–91°C.

Analyse für $C_{20}H_{20}O_4$:

| | | | |
|---|---|---|---|
| Ber. | C 74,06% | H 6,22% | O 19,73% |
| Gef. | C 74,04% | H 6,17% | O 19,72% |

Beispiel 106:

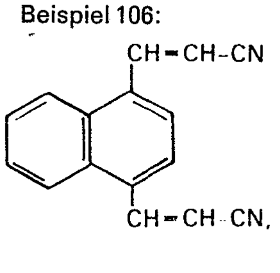

analog Beispiel 105 unter Verwendung von 1,66 g (31,25 mMol) Acrylnitril; während 2 Std. bei 120°C gerührt. Das Rohprodukt wird im Soxhlet mit Diäthyläther extrahiert und aus Toluol/Tetrahydrofuran umkristallisiert. Man erhält 0,5 g (17% d.Th.) hellgelber Kristalle vom Fp. 291–292°C.

Analyse für $C_{16}H_{10}N_2$:

| | | | |
|---|---|---|---|
| Ber. | C 83,46% | H 4,38% | N 12,17% |
| Gef. | C 83,08% | H 4,44% | N 12,28% |

Beispiel 107:

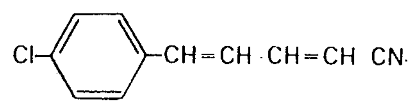

0,224 g (1 mMol) Palladiumacetat, 20,1 g (100 mMol) 4-Chlorzimtsäurechlorid, 6,63 g (125 mMol) Acrylnitril und 18,53 g (100 mMol) Tri-n-butylamin werden in 100 ml p-Xylol während 2 Stunden bei 120°C gerührt. Das Rohprodukt wird auf Kieselgel in Toluol chromatographiert und dann aus n-Hexan umkristallisiert. Man erhält 0,9 g (5% d.Th.) gelber Kristalle vom Fp. 83,7°C.

Analyse für $C_{11}H_8ClN$:

| | | | |
|---|---|---|---|
| Ber. | C 69,66% | H 4,25% | N 7,38% |
| Gef. | C 69,92% | H 4,24% | N 7,49% |

Beispiel 108:

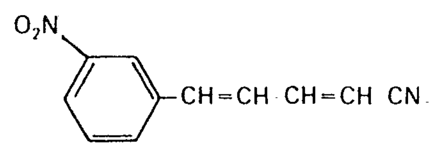

0,112 g (0,5 mMol) Palladiumacetat, 10,58 g (50 mMol) 3-Nitrozimtsäurechlorid, 3,31 g (62,5 mMol) Acrylnitril und 9,27 g (50 mMol) Tri-n-butylamin in 50 ml p-Xylol werden während 2 Std. bei 120°C gerührt. Das Rohprodukt wird auf Kieselgel in Toluol chromatographiert und dann aus Tetrachlorkohlenstoff umkristallisiert. Man erhält

3,6 g (36% d.Th.) hellgelber Kristalle vom Fp. 107,1°C.

Analyse für $C_{11}H_8N_2O_2$:

Ber. C 66,00% H 4,03% N 14,00%
Gef. C 65,69% H 3,93% N 14,05%

Beispiel 109:
1-(2-Äthoxycarbonyl)-vinylnaphthalin.
0,2244 g ($10^{-3}$ Mol) Palladiumacetat, 15,05 g (0,1 Mol) Naphthalin-1-carbonsäurechlorid, 13,54 ml (0,125 Mol) Acrylsäureäthylester und 23,95 ml (0,1 Mol) Tri-n-butylamin in 200 ml p-Xylol werden 1 Std. bei 120°C gerührt. Das Rohprodukt wird im Vakuum destilliert. Man erhält 15,6 g (69% d.Th.) einer farblosen Flüssigkeit (destilliert bei 143-147°C/106 Pa).

Analyse für $C_{15}H_{14}O_2$:

Ber. C 79,62% H 6,24% O 14,14%
Gef. C 79,24% H 6,39% O 14,27%

Beispiel 110:
Die untenstehend angegebenen Mengen Palladiumacetat, 5,77 ml (50 mMol) Benzoylchlorid, 5,42 ml (50 mMol) Acrylsäureäthylester und 7,53 ml (50 mMol) N-Benzyldimethylamin in 100 ml p-Xylol werden 1,5-6 Std. bei 130°C gerührt.
Die Ausbeuten an Zimtsäureäthylester sind in Tabelle 1 angegeben.

Tabelle 1

| Palladium-acetat Mol.%1) | Reaktions-dauer h | Ausbeute % d.Th. | Umsatz-zahl2) |
|---|---|---|---|
| 0,05 | 1,5 | 80 | 1 600 |
| 0,02 | 2 | 76 | 3 800 |
| 0,01 | 2,5 | 70 | 7 030 |
| 0,005 | 6 | 53 | 10 580 |

1) bezogen auf das Benzoylchlorid
2) Umsatzzahl = $\frac{\text{Mol gebildetes Produkt}}{\text{Mol eingesetzter Katalysator}}$

Beispiel 111:
Die in der untenstehenden Tabelle 2 angegebenen Mengen Palladiumacetat, 6,74 g (40 mMol) 4-Formylbenzoylchlorid, 2,63 ml (40 mMol) Acrylnitril und 5,12 ml (40 mMol) N-Äthylmorpholin in 80 ml p-Xylol werden 5 Std. bei 120°C gerührt. Die Ausbeuten an 4-Formylzimtsäurenitril sind in Tabelle 2 angegeben.

Tabelle 2

| Palladiumacetat Mol.% | Ausbeute % d.Th. | Umsatzzahl |
|---|---|---|
| 0,1 | 84 | 840 |
| 0,04 | 64 | 1 600 |
| 0,033 | 74 | 2 200* |
| 0,02 | 31 | 1 550 |
| 0,01 | 10 | 1 000 |

Tabelle 2 (Fortsetzung)

* mit 44 mMol Acrylnitril in 12 Std.

Beispiel 112:
0,095 g (2,5 × $10^{-4}$ Mol) Diacetatobipyridylpalladium(II), 2,88 ml (25 mMol) Benzoylchlorid, 3,39 ml (31,25 mMol) Acrylsäureäthylester und 5,96 ml (25 mMol) Tri-n-butylamin in 50 ml p-Xylol werden während 3 Std. bei 120°C gerührt. Nach der Aufarbeitung wie in den vorangehenden Beispielen beschrieben erhält man 3,78 g (86% d.Th.) Zimtsäureäthylester.

Beispiel 113:
3,91 mg (1,6 × $10^{-5}$ Mol) Palladiumacetat, 6,74 g (40 mMol) 4-Formylbenzylchlorid, 2,63 ml (40 mMol) Acrylnitril und 4,41 ml (40 mMol) N-Methylmorpholin werden in 80 ml p-Xylol während 5 Stunden gerührt. Nach der Aufarbeitung wie in den vorangehenden Beispielen beschrieben erhält man 0,53 g (8% d.Th.) 4-Formylzimtsäurenitril.

Beispiel 114:
Zimtsäureäthylester wird nach den oben beschriebenen Verfahren hergestellt, wobei unterschiedliche Basen und die folgenden Reaktionskomponenten eingesetzt werden: 2,24 mg ($10^{-5}$ Mol) Palladiumacetat, 5,77 ml (50 mMol) Benzoylchlorid, 5,42 ml (50 mMol) Acrylsäureäthylester und 50 mMol N-Benzyldiäthylamin oder N-(3-Chlorbenzyl)-dimethylamin in 100 ml p-Xylol. Das Reaktionsgemisch wird während 4 Stunden bei 130°C gerührt. Man erhält den Zimtsäureäthylester in einer Ausbeute von 67% d.Th. bei Verwendung von N-Benzyldiäthylamin und 56% d.Th. bei Verwendung von N-(3-Chlorbenzyl)-dimethylamin.

Beispiel 115:
0,2877 g (0,5 mMol) Bis-(Dibenzylidenaceton)Palladium(O), 9,98 g (50 mMol) 2-Methyl-5-nitrobenzoylchlorid, 3,31 g (62,5 mMol) Acrylnitril und 17,68 g (50 mMol) Tris-(2-Äthylhexyl)-amin in 100 ml Cyclopentanon werden während 1,5 Std. bei 100°C gerührt. Das Rohprodukt wird auf Kieselgel in Methylenchlorid chromatographiert und dann aus Cyclohexan/Tetrachlorkohlenstoff umkristallisiert. Man erhält 2,1 g (24% d.Th.) 2-Methyl-5-nitrozimtsäurenitril in Form weisser Kristalle; Fp. 113,2°C.

Beispiel 116:
0,152 g (0,5 mMol) Bis-(Acetylacetonato)Palladium(II), 10,53 g (50 mMol) 3,4-Dichlorbenzoylchlorid, 7,5 g (75 mMol) Acrylsäureäthylester und 17,65 g (50 mMol) Tri-n-octylamin in 100 ml Oxalsäurediäthylester werden während 2,5 Std. bei 120°C gerührt. Das Rohprodukt wird im Vakuum destilliert und dann aus Methanol umkristallisiert. Man erhält 1,4 g (11% d.Th.) 3,4-Dichlorzimtsäureäthylester; Fp. 56,0°C.

Beispiel 117:

0,0792 g (5 mMol) Palladium(II)cyanid, 10,03 g (50 mMol) 3,5-Dimethoxybenzoylchlorid, 6,26 g (62,5 mMol) Acrylsäureäthylester und 6,46 g (50 mMol) N-Äthyldiisopropylamin in 100 ml Benzonitril werden während 70 Minuten bei 140°C gerührt. Das Rohprodukt wird im Vakuum destilliert. Nach der Aufarbeitung erhält man 5,83 g (49% d.Th.) 3,5-Dimethoxyzimtsäureäthylester als farblose Flüssigkeit.

Beispiel 118:

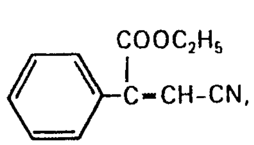

Zu 0,0561 g (0,25 mMol) Palladiumacetat in 50 ml p-Xylol werden unter Argon 3,51 g (25 mMol) Benzoylchlorid, 3,13 g (25 mMol) β-Cyanacrylsäureäthylester und 3,38 g (25 mMol) N-Benzyldimethylamin zugegeben, und das Gemisch wird während 7 Std. bei 130°C gerührt. Das Rohprodukt wird im Vakuum destilliert; Sdp. 92–107°C/13 Pa. Man erhält 2,3 g (46% d.Th.) eines Isomerengemisches des Zimtsäurenitril-β-carbonsäureäthylesters, das aus E- und Z-Isomeren besteht.

Beispiel 119:

Zu 80 ml Anisol werden 0,0018 g (0,008 mMol) Palladiumacetat, 6,74 g (40 mMol) 4-Formylbenzoylchlorid, 3,29 g (50 mMol) Acrylnitril und 5,12 ml (40 mMol) N-Äthylmorpholin zugegeben. Das Gemisch wird während 12 Std. bei 120°C unter Argon gerührt. Das Rohprodukt wird im Vakuum destilliert und anschliessend aus Isopropanol umkristallisiert. Man erhält 3,2 g (52% d.Th.) 4-Formylzimtsäurenitril in Form weisser Kristalle.

Beispiel 120:

Zu 0,561 g (2,5 mMol) Palladiumacetat in 500 ml p-Xylol werden 54,88 g (0,25 Mol) 4-Brombenzoylchlorid, 20,81 ml (0,31 Mol) Acrylnitril und 37,65 ml (0,25 Mol) N-Benzyldimethylamin zugegeben. Das Gemisch wird bei 120°C während 1,5 Std. gerührt. Das Rohprodukt wird destilliert und aus Cyclohexanon umkristallisiert. Man erhält 35 g (67% d.Th.) 4-Bromzimtsäurenitril in Form weisser Blättchen; Fp. 105,7°C.

Analyse für $C_9H_4NBr$:

| | | | | |
|---|---|---|---|---|
| Ber. | C 51,96% | H 2,91% | N 6,73% | Br 38,40% |
| Gef. | C 51,93% | H 3,01% | N 6,69% | Br 38,35% |

Beispiel 121:

Unter Verwendung verschiedener Katalysatoren wird nach der in den vorangehenden Beispielen beschriebenen arbeitsweise Zimtsäureäthylester wie folgt hergestellt: Zu 50 ml p-Xylol gibt man unter Argon 0,1 g Palladiummetall bzw. je 0,25 mMol der unten aufgeführten Katalysatoren, 2,89 ml (25 mMol) Benzoylchlorid, 2,71 ml (25 mMol) Acrylsäureäthylester und 3,77 ml (25 mMol) N-Benzyldimethylamin und rührt das Reaktionsgemisch während 2 Std. bei 130°C. Nach der Aufarbeitung erhält man den Zimtsäureäthylester in den unten angegebenen Ausbeuten.

| Katalysator | Ausbeute |
|---|---|
| 0,0613 g (0,25 mMol) $[Pd(NH_3)_4]Cl_2$ | 80% d.Th. |
| 0,081 g (0,25 mMol) Bis-(Cyclohexylisonitril)Palladium(O) | 80% d.Th. |
| 0,0833 g (0,25 mMol) $PdCl_2[OS(CH_3)_2]_2$ | 69% d.Th. |
| 0,0958 g (0,25 mMol) $PdCl_2(NC\text{-}Phenyl)_2$ | 78% d. Th. |
| 0,1 g Palladiummetall | 9% d.Th. |

## Patentansprüche:

1. Verfahren zur Herstellung von Verbindungen der Formel I

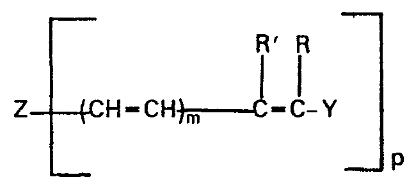 (I),

worin

m Null oder die Zahl 1,

p die Zahl 1 oder 2,

Z bei p = 1 gegebenenfalls substituiertes Phenyl oder Naphthyl und bei p = 2 gegebenenfalls substituiertes Phenylen oder Naphthylen,

R Wasserstoff, $C_{1-4}$-Alkyl, $-CH_2COOR''$ oder $-CH_2CH_2CN$ und

R′ Wasserstoff oder bei p = 1 und m = Null auch $C_{1-4}$-Alkyl, -CN oder -COOR″ bedeuten, wobei mindestens eines von R und R′ Wasserstoff sein muss,

Y -CN, -COOR″, $-CON(R'')_2$ oder -COR″ und die R″ unabhängig voneinander $C_1$–$C_{12}$-Alkyl oder Phenyl darstellen,

dadurch gekennzeichnet, dass man eine Verbindung der Formel II

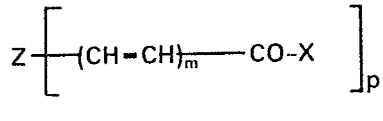 (II),

worin Z, m und p die unter Formel I angegebene Bedeutung haben und X Chlor, Brom oder Jod darstellt, in Gegenwart einer Base und unter Zusatz von Palladium-Metall oder von Palladiumverbindungen, die unter den Reaktionsbedingungen phosphorfreie labile Palladium(O)Verbindungen

bilden, als Katalysator mit einer Verbindung der Formel III oder bei p = 2, auch einem Gemisch von zwei verschiedenen Verbindungen der Formel III

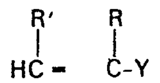 (III),

worin für R, R′ und Y das unter Formel I Angegebene gilt, umsetzt.

2. Verfahren nach Anspruch 1, dadurch gekennzeichnet, dass man eine Verbindung der Formel II verwendet, worin X Chlor darstellt.

3. Verfahren nach Anspruch 1, dadurch gekennzeichnet, dass man als Palladiumverbindung $PdCl_2$,Pd $Br_2$ $Pd(OOCCH_3)_2$,$Pd(CH_3COCHCOCH_3)_2$, $Pd(OOCCH_3)_2$(2,2′-Bipyridyl), $PdCl_2$(NC-Phenyl)$_2$, Bis-(Dibenzylidenaceton)-Palladium(O) oder Bis-(Cyclohexylisonitril)Palladium(O) verwendet.

4. Verfahren nach Anspruch 1, dadurch gekennzeichnet, dass man als Palladiumverbindung $PdCl_2$, Palladiumacetat oder Bis-(Dibenzylidenaceton)Palladium(O) verwendet.

5. Verfahren nach Anspruch 1, dadurch gekennzeichnet, dass man die Umsetzung bei einer Temperatur zwischen 0 und 200°C und in Gegenwart eines gegenüber den Reaktionspartnern inerten organischen Lösungsmittels vornimmt.

6. Verfahren nach Anspruch 5, dadurch gekennzeichnet, dass man als Lösungsmittel Anisol, Xylole oder Toluol verwendet.

7. Verfahren nach Anspruch 1, dadurch gekennzeichnet, dass man als Base eine Verbindung der Formel VI

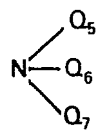 (VI)

verwendet, worin $Q_5$ 4-Chlorbenzyl, 3-Methylbenzyl, 3-Methoxybenzyl oder Benzyl und $Q_6$ und $Q_7$ je Alkyl mit 1-4 C-Atomen bedeuten, oder worin $Q_5$, $Q_5$ und $Q_7$ je Alkyl mit 3-12 C-Atomen darstellen.

8. Verfahren nach Anspruch 1, dadurch gekennzeichnet, dass man als Base N-Benzyldimethylamin, N-Äthylmorpholin oder Tri-n-butylamin verwendet.

9. Verfahren nach Anspruch 1, dadurch gekennzeichnet, dass man den Katalysator in einer Menge von 0,001 bis 3 Mol%, bezogen auf die Verbindung der Formel II, verwendet.

10. Verfahren nach Anspruch 1, dadurch gekennzeichnet, dass man als Säurehalogenid eine Verbindung der Formel IIa, IIb oder IIc

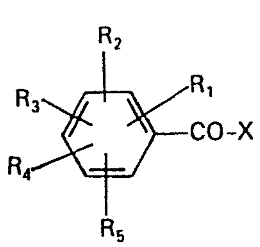 (IIa),

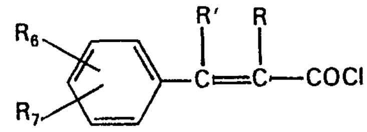 (IIb),

oder

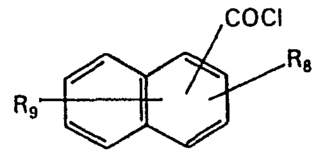 (IIc),

Iso- oder Terephthalsäuredichlorid, 1,4- oder 2,6-Naphthalindicarbonsäuredichlorid verwendet, wobei X Chlor, eines von R und R′ Wasserstoff und das andere Wasserstoff oder Methyl oder R Wasserstoff und R′ -COOAlkyl mit 1-4 C-Atomen im Alkyl, $R_1$ Wasserstoff, Cl, Br, F, J, Formyl, Methyl, Methoxy, Chlormethyl, Cyano, Nitro, $-OCOCH_3$, $-COOCH_3$ oder Phenyl, $R_2$ Wasserstoff, Cl, F, Methyl oder Methoxy, $R_3$ Wasserstoff, Cl, F oder Methoxy und $R_4$ und $R_5$ je Wasserstoff, oder bei $R_1$ bis $R_3$ = Cl oder F, ebenfalls Cl oder F bedeuten, $R_6$ Wasserstoff, Chlor oder Nitro, $R_7$ Wasserstoff, $R_8$ Methyl und insbesondere Wasserstoff und $R_9$ Wasserstoff darstellen.

11. Verfahren nach Anspruch 1, dadurch gekennzeichnet, dass man eine Verbindung der Formel III verwendet, worin R und R′ Wasserstoff und Y -CN, -COR″, -COOR″ oder -CON(R″)$_2$ mit R″ = je Methyl oder Äthyl, R Wasserstoff, R′ Methyl und Y -CN, $-COOCH_3$, $-COOC_2H_5$, $-CON(CH_3)_2$ oder $-CON(C_2H_5)_2$, R Wasserstoff, R′ und Y je -CN, $-COOCH_3$, oder $-COOC_2H_5$, R′ Wasserstoff, R Methyl und Y -CN, $-COOCH_3$, $-COOC_2H_5$, $-CON(CH_3)_2$ oder $-CON(C_2H_5)_2$, R′ Wasserstoff, R $-CH_2COOCH_3$ und Y $-COOCH_3$ oder R′ Wasserstoff, R $-CH_2CH_2CN$ und Y -CN sind.

12. Verfahren nach Anspruch 1, dadurch gekennzeichnet, dass man als Verbindung der Formel II 4-Formylbenzoylchlorid, 4-Brombenzoylchlorid und insbesondere Benzoylchlorid und als Verbindung der Formel III Acrylsäureäthylester, Acrylnitril oder N,N-Diäthylacrylamid verwendet.

## Claims

1. A process for the preparation of a compound of the formula I

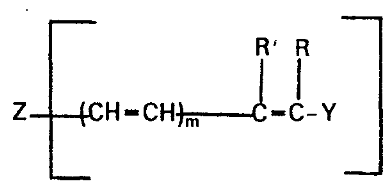 (I),

in which m is zero or 1, p is 1 or 2, Z is substituted or unsubstituted phenyl or naphthyl if p = 1, and substituted or unsubstituted phenylene or naphthylene if p = 2, R is hydrogen, $C_{1-4}$-alkyl, $-CH_2COOR''$ or $-CH_2CH_2CN$ and R′ is hydrogen,

or, if p = 1 and m = zero, can also be $C_{1-4}$-alkyl, -CN or -COOR″, but at least one of R and R′ must be hydrogen, Y is -CN, -COOR″, $-CON(R'')_2$ or -COR″ and the radicals R″, independently of one another, are $C_1$-$C_{12}$-alkyl or phenyl, which comprises reacting a compound of the formula II

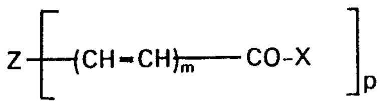
(II),

in which Z, m and p are as defined for formula I and X is chlorine, bromine or iodine, with a compound of the formula III or if desired, where p = 2, with a mixture of two different compounds of the formula III

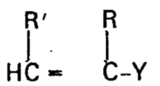
(III),

in which R, R′ and Y are as defined for formula I, in the presence of a base and with the addition, as a catalyst, of palladium metal or of a palladium compound which under the reaction conditions forms a phosphorus-free labile palladium(O) compound.

2. A process according to claim I, wherein a compound of the formula II, in which X is chlorine, is used.

3. A process according to claim 1, wherein the palladium compound used is $PdCl_2$, $PdBr_2$, $Pd(OOCCH_3)_2$, $Pd(CH_3COCHCOCH_3)_2$, $Pd(OOCCH_3)_2$(2,2′-bipyridyl), $PdCl_2$(NC-phenyl)$_2$, bis-(dibenzylidine-acetone)-palladium(O) or bis-(cyclohexylisonitrile)-palladium(O).

4. A process according to claim 1, wherein the palladium compound used is $PdCl_2$, palladium acetate or bis-(dibenzylidene-acetone)-palladium(O).

5. A process according to claim 1, wherein the reaction is carried out at a temperature of between 0 and 200°C and in the presence of an organic solvent which is inert towards the reactants.

6. A process according to claim 5, wherein the solvent used is anisole, a xylene, or toluene.

7. A process according to claim 1, wherein the base used is a compound of the formula VI

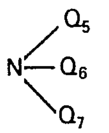
(VI)

in which $Q_5$ is 4-chlorobenzyl, 3-methylbenzyl, 3-methoxybenzyl or benzyl and $Q_6$ and $Q_7$ are each alkyl having 1-4 C atoms, or in which $Q_5$, $Q_5$ and $Q_7$ are each alkyl having 3-12 C atoms.

8. A process according to claim 1, wherein the base used is N-benzyldimethylamine, N-ethylmorpholine or tri-n-butylamine.

9. A process according to claim 1, wherein the amount of catalyst used is from 0.001 to 3 mol %, based on the compound of the formula II.

10. A process according to claim 1, wherein the acid halide used is a compound of the formula IIa, IIb or IIc

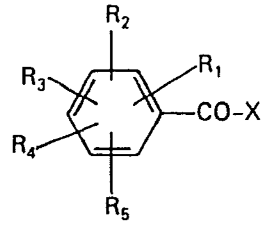
(IIa),

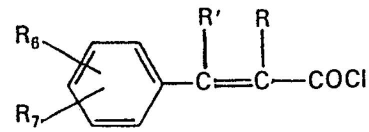
(IIb),

or

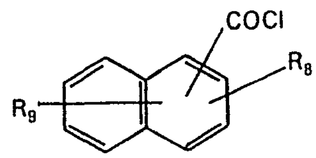
(IIc),

in which X is chlorine, one of R and R′ is hydrogen and the other is hydrogen or methyl, or R is hydrogen and R′ is -COO-alkyl having 1-4 C atoms in the alkyl moiety, $R_1$ is hydrogen, Cl, Br, F, I, formyl, methyl, methoxy, chloromethyl, cyano, nitro, $-OCOCH_3$, $-COOCH_3$ or phenyl, $R_2$ is hydrogen, Cl, F, methyl or methoxy, $R_3$ is hydrogen, Cl, F or methoxy and $R_4$ and $R_5$ are each hydrogen, or, if $R_1$, $R_2$ and $R_3$ are Cl or F, are also Cl or F, $R_6$ is hydrogen, chlorine or nitro, $R_7$ is hydrogen, $R_8$ is methyl and especially hydrogen and $R_9$ is hydrogen, or the acid halide used is isophthalic acid dichloride, terephthalic acid dichloride, 1,4-naphthalenedicarboxylic acid dichloride or 2,6-naphthalenedicarboxylic acid dichloride.

11. A process according to claim 1, wherein a compound of the formula III is used, in which R and R′ are hydrogen and Y is -CN, -COR″, -COOR″ or $-CON(R'')_2$, each R″ being methyl or ethyl, or R is hydrogen, R′ is methyl and Y is -CN, $-COOCH_3$, $-COOC_2H_5$, $-CON(CH_3)_2$ or $-CON(C_2H_5)_2$, or R is hydrogen and R′ and Y are each -CN, $-COOCH_3$ or $-COOC_2H_5$, or R′ is hydrogen, R is methyl and Y is -CN, $-COOCH_3$, $-COOC_2H_5$, $-CON(CH_3)_2$ or $-CON(C_2H_5)_2$, or R′ is hydrogen, R is $-CH_2COOCH_3$ and Y is $-COOCH_3$, or R′ is hydrogen, R is $-CH_2CH_2CN$ and Y is -CN.

12. A process according to claim 1, wherein 4-formylbenzoyl chloride, 4-bromobenzoyl chloride or especially benzoyl chloride is used as the compound of the formula II and ethyl acrylate, acrylonitrile or N,N-diethylacrylamide is used as the compound of the formula III.

**Revendications**

1. Procédé de préparation de composés répondant à la formule I

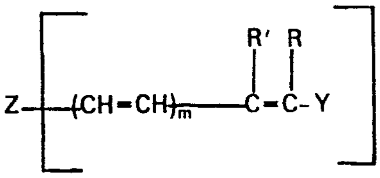

(I),

dans laquelle
m représente un nombre égal à 0 ou à 1,
p représente un nombre égal à 1 ou à 2,
Z représente, dans le cas où p est égal à 1, un radical phényle ou naphtyle éventuellement substitué et, dans le cas où p est égal à 2, un radical phénylène ou naphtylène éventuellement substitué,
R représente l'hydrogène, un alkyle en $C_1$-$C_4$, -$CH_2COOR''$ ou -$CH_2CH_2CN$,
R' représente l'hydrogène ou, dans le cas où p est égal à 1 et m à 0, peut aussi représenter un alkyle en $C_1$-$C_4$, -CN ou -COOR'', au moins un des symboles R et R' devant représenter l'Hydrogène,
Y représente -CN, -COOR'', -$CON(R'')_2$ ou -COR'' et
les R'' représentent chacun, indépendamment les uns des autres, un alkyle en $C_1$-$C_{12}$ ou un phényle, procédé caractérisé en ce qu'on fait réagir un composé répondant à la formule II

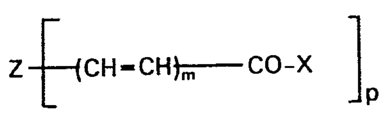

(II),

dans laquelle Z, m et p ont les significations qui ont été données au-dessous de la formule I et X représente le chlore, le brome ou l'iode, en présence d'une base et également, comme catalyseur, de palladium métallique ou de composé du palladium qui, dans les conditions de la réaction forment des composés du palladium(O) labiles non phosphorés, avec un composé ou, dans le cas où p est égal à 2, avec un mélange de deux composés répondant à la formule III

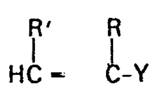

(III),

dans laquelle R, R' et Y ont les significations qui viennent d'être données à propos de la formule I.

2. Procédé selon la revendication 1 caractérisé en ce qu'on utilise un composé de formule II dans lequel X représente le chlore.

3. Procédé selon la revendication 1 caractérisé en ce qu'on utilise, comme composé du palladium, $PdCl_2$, $PdBr_2$, $Pd(OOCCH_3)_2$, $Pd(CH_3COCHCOCH_3)_2$, $Pd(OOCCH_3)_2$(bipyridyl-2,2'), $PdCl_2$ (NC-phényl)$_2$, le bis-(dibenzylidène-acétone)-palladium(O) ou le bis-(cyclohexylisonitrile)-palladium(O).

4. Procédé selon la revendication 1 caractérisé en ce qu'on utilise, comme composé du palladium, $PdCl_2$, l'acétate de palladium ou le bis-(dibenzylidèneacétone)palladium(O).

5. Procédé selon la revendication 1 caractérisé en ce qu'on effectue la réaction à une température comprise entre 0 et 200°C et en présence d'un solvant organique inerte à l'égard des corps prenant part à la réaction.

6. Procédé selon la revendication 5 caractérisé en ce qu'on utilise, comme solvant, l'anisole, des xylènes ou le toluène.

7. Procédé selon la revendication 1 caractérisé en ce qu'on utilise, comme base, un composé répondant à la formule VI

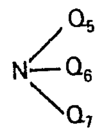

(VI)

dans laquelle $Q_5$ représente un radical chloro-4 benzyle, méthyl-3 benzyle, méthoxy-3 benzyle ou benzyle, et $Q_6$ et $Q_7$ représentent chacun un radical alkyle contenant de 1 à 4 atomes de carbone, ou dans laquelle $Q_6$, $Q_6$ et $Q_7$ représentent chacun un radical alkyle en $C_3$-$C_{12}$.

8. Procédé selon la revendication 1 caractérisé en ce qu'on utilise, comme base, la N-benzyldiméthylamine, la N-éthyl-morpholine ou la tri-n-butylamine.

9. Procédé selon la revendication 1 caractérisé en ce qu'on utilise le catalyseur en une quantité de 0,001 à 3% en moles par rapport au composé de formule II.

10. Procédé selon la revendication 1 caractérisé en ce qu'on utilise, comme halogénure d'acide, un composé répondant à l'une des formules IIa, IIb et IIc:

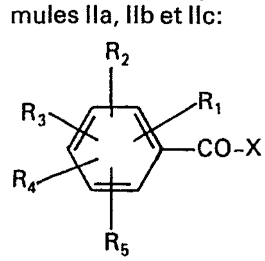

(IIa),

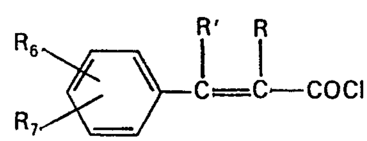

(IIb),

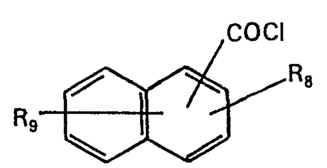

(IIc),

le dichlorure de l'acide isophtalique ou de l'acide téréphtalique, le dichlorure de l'acide naphtalène-carboxylique-1,4 ou -2,6, les divers symboles présents dans les formules précédentes ayant les significations suivantes: X représente le chlore, l'un des symboles R et R' représente

l'hydrogène et l'autre repésente l'hydrogène ou un méthyle, ou encore R représente l'hydrogéne et R′ un radical alcoxycarbonyle dont la partie alkyle contient de 1 à 4 atomes de carbone, $R_1$ représente l'hydrogène, Cl, Br, F, I ou un radical formyle, méthyle, méthoxy, chlorométhyle, cyano, nitro, $-OCOCH_3$, $-COOCH_3$ ou phényle, $R_2$ représente l'hydrogène, Cl, F ou un radical méthyle ou méthoxy, $R_3$ représente l'hydrogène, Cl, F ou un radical méthoxy, $R_4$ et $R_5$ représentent chacun l'hydrogène ou, lorsque $R_1$ à $R_3$ représentent Cl ou F, $R_4$ et $R_5$ peuvent également représenter chacun Cl ou F, $R_6$ représente l'hydrogène, le chlore ou un groupe nitro, $R_7$ représente l'hydrogène, $R_8$ représente un radical méthyle ou, plus particulièrement, l'hydrogène, et $R_9$ représente l'hydrogène.

11. Procédé selon la revendication 1 caractérisé en ce qu'on utilise un composé de formule III dans lequel R et R′ représente chacun l'hydrogène et Y représente un radical -CN, -COR″, -COOR″ ou $-CON(R'')_2$, où les R″ représentent chacun un méthyle ou un éthyle; R représente l'hydrogène, R′ représente un méthyle et Y représente un radical -CN, $-COOCH_3$, $-COOC_2H_5$, $-CON(CH_3)_2$ ou $-CON(C_2H_5$; R représente l'hydrogène, R′ et Y représentent chacun -CN, $-COOCH_3$ ou $-COOC_2H_5$; R′ représente l'hydrogène, R représente un méthyle et Y représente un radical -CN, $-COOCH_3$, $-COOC_2H_5$, $-CON(CH_3)_2$ ou $-CON(C_2H_5)_2$; R′ représente l'hydrogène, R un radical $-CH_2COOCH_3$ et Y un radical $-COOCH_3$; ou R′ représente l'hydrogène, R un radical $-CH_2CH_2CN$ et Y un radical -CN.

12. Procédé selon la revendication 1 caractérisé en ce qu'on utilise, comme composé de formule II, le chlorure de formyl-4 benzoyle, le chlorure de bromo-4 benzoyle ou, plus spécialement, le chlorure de benzoyle, et, comme composé de formule III, l'acrylate d'éthyle, l'acrylonitrile ou le N,N-diéthyl-acrylamide.